(19) Europäisches Patentamt
European Patent Office
Office européen des brevets

(11) Numéro de publication: 0 010 458
B1

(12) FASCICULE DE BREVET EUROPEEN

(45) Date de publication du fascicule du brevet: 10.08.83

(21) Numéro de dépôt: 79400585.0

(22) Date de dépôt: 23.08.79

(51) Int. Cl.³: C 07 D 519/04,
A 61 K 31/475

# (54) Composés bis-indoliques, compositions pharmaceutiques les contenant, procédé pour leur préparation.

(30) Priorité: 24.08.78 FR 7824568
24.08.78 FR 7824569
06.02.79 FR 7902981

(43) Date de publication de la demande:
30.04.80 Bulletin 80/9

(45) Mention de la délivrance du brevet:
10.08.83 Bulletin 83/32

(84) Etats contractants désignés:
BE CH DE GB IT NL

(56) Documents cités:
néant

(73) Titulaire: ANVAR Agence Nationale de Valorisation de la Recherche
13, rue Madeleine Michelis
F-92522 Neuilly-sur-Seine (FR)

(72) Inventeur: Potier, Pierre
5, rue de la Fontaine
F-78390 Bois d'Arcy (FR)
Inventeur: Langlois née Petit, Nicole
32, rue de Gometz
F-91440 Bures S. Yvette (FR)
Inventeur: Langlois, Yves
32, rue de Gometz
F-91440 Bures S. Yvette (FR)
Inventeur: Andriamialisoa, Ratremaniaina Zo
Les Millepertuis Batiment Al
91440 Les Ulis (FR)
Inventeur: Mangeney, Pierre
10, rue de l'Ingénieur Keller
75015 Paris (FR)

(74) Mandataire: Corre, Jacques Denis Paul et al,
Cabinet Regimbeau 26, Avenue Kléber
F-75116 Paris (FR)

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 010 458 B1

Courier Press, Leamington Spa, England.

# Composés bis-indoliques, compositions pharmaceutiques les contenant, procédé pour leur préparation

La présente invention concerne de nouveaux composés bis-indoliques et un procédé de préparation de ces composés ainsi que leur application en tant que médicaments.

Il est connu depuis un certain temps que des alcaloïdes naturels, tels que la vinblastine ou la vincristine qui répondent à la formule

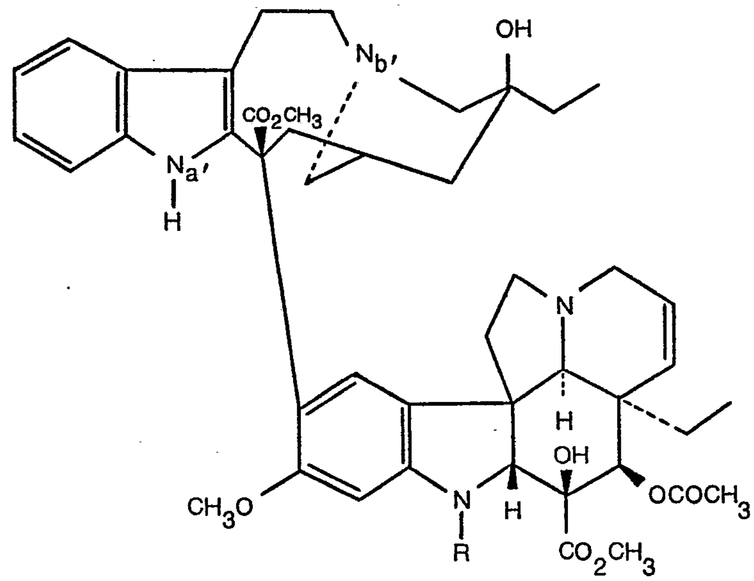

$R = CH_3$ : vinblastine, $R = CHO$ : vincristine) et qui peuvent être isolés de plusieurs espèces de *Catharanthus*, en particulier de *C. roseus* présentent des propriétés anti-tumorales. Compte tenu du fait que ces alcaloïdes ne sont présents qu'en très faible quantité dans la plante, on a cherché à préparer des dérivés plus actifs de ces composés, voir ainsi les BSM 5487 M, 6668 M, le brevet français 2218 095. On a également proposé, voir les brevets français 74 43221 et 77 11081, des procédés de synthèse ouvrant la voie à de nouveaux composés de structure identique mais diversement substitués.

Néanmoins dans l'ensemble de la technique antérieure les composés proposés conservaient toujours le squelette de base de la vinblastine.

La présente invention a pour objet des composés chimiques nouveaux possédant notamment une activité antitumorale et présentant un squelette de base différent de celui de la vinblastine.

Il s'agit de composés de formule:

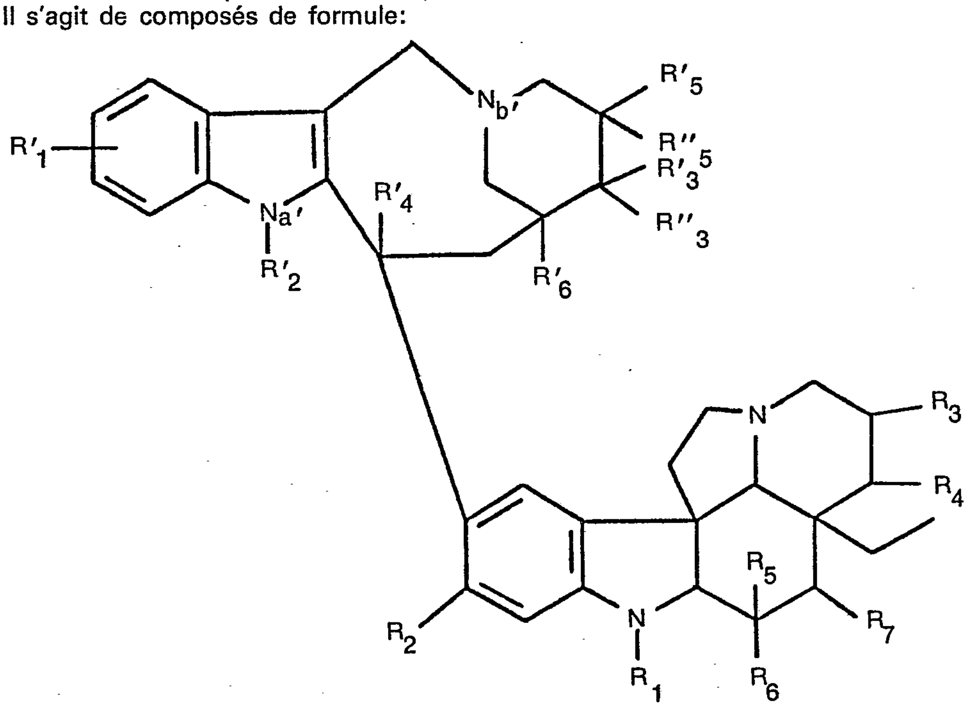

(I)

dans laquelle:

$R'_1$ représente un atome d'hydrogène ou un radical alcoxy, acyle, formyle ou halogénoacycle,

$R'_2$ représente un atome d'hydrogène ou un radical alcoyle,

$R'_3$ et $R''_3$ identiques ou différentes, représentent un atome d'hydrogène ou un radical hydroxyle, alcanoyloxyle, ou bien $R'_3$ et $R''_3$ forment ensemble un groupement carbonyle, ou bien $R'_3$ et $R'_5$ forment ensemble un pont époxy ou une double liaison.

$R'_4$ représente un atome d'hydrogène ou un radical alcoyloxycarbonyle, hydroxyméthyle, alcanoyloxyméthyle ou acétamido,

$R'_5$ et $R''_5$ identiques ou différents, représentent un atome d'hydrogène ou un radical hydroxyle, alcanoyloxyle, éthyle ou hydroxy-2 éthyle,

$R'_6$ représente un atome d'hydrogène ou un radical éthyle, hydroxy-2 éthyle ou acétyle,

$R_1$ représente un atome d'hydrogène ou un radical alcoyle, formyle ou acyle,

$R_2$ représente un atome d'hydrogène ou un radical alcoxy,

$R_3$ représente un atome d'hydrogène ou un radical hydroxyle ou alcanoyloxyle ou bien, $R_3$ et $R_4$ forment ensemble un pont époxy ou une double liaison,

$R_4$ représente un atome d'hydrogène ou un radical hydroxyle, alcanoyloxyle ou bien, $R_4$ et $R_5$ forment ensemble un pont époxy,

$R_6$ représente un radical alcoyloxycarbonyle, hydrazido, acétamido, hydroxyméthyle ou alcanoyloxyméthyle,

$R_5$ et $R_7$ représentent un atome d'hydrogène ou un radical hydroxyle ou alcanoyloxyle,

ainsi que leurs sels d'addition avec les acides et leurs sels d'ammonium quaternaire et les 12-chloro dérivés.

Les radicaux alcoyles mentionnés dans la présente description sont des radicaux alcoyles inférieurs droits ou ramifiés ayant de 1 à 5 atomes de carbone, comme les radicaux méthyle ou éthyle.

Les radicaux alcoxy mentionnés dans la présente description sont des radicaux alcoxy inférieurs correspondant aux radicaux alcoyles précédents, c'est-à-dire par exemple les radicaux méthoxy et éthoxy.

Les radicaux acyles cités dans la présente description sont les radicaux acyles provenant des acides carboxyliques inférieurs, saturés ou non saturés, tels que les radicaux acétyle ou propionyle.

De même, les radicaux alcanoyloxy sont des radicaux correspondant aux radicaux acyles précédents comme le radical acétyloxy.

Les radicaux alcoyloxycarbonyles sont des radicaux dans lesquels la partie alcoyle correspond à la définition préférée donnée précédemment, par exemple le radical méthoxycarbonyle.

Parmi les sels d'addition et les sels d'ammonium quaternaire, on préparera de préférence les sels non-toxiques pharmaceutiquement acceptables tels que les sels d'acide inorganique comme l'acide chlorhydrique ou d'acide organique comme l'acide acétique, l'acide propionique, l'acide succinique ou l'acide tartrique.

L'invention concerne plus particulièrement les composés de formule Ia

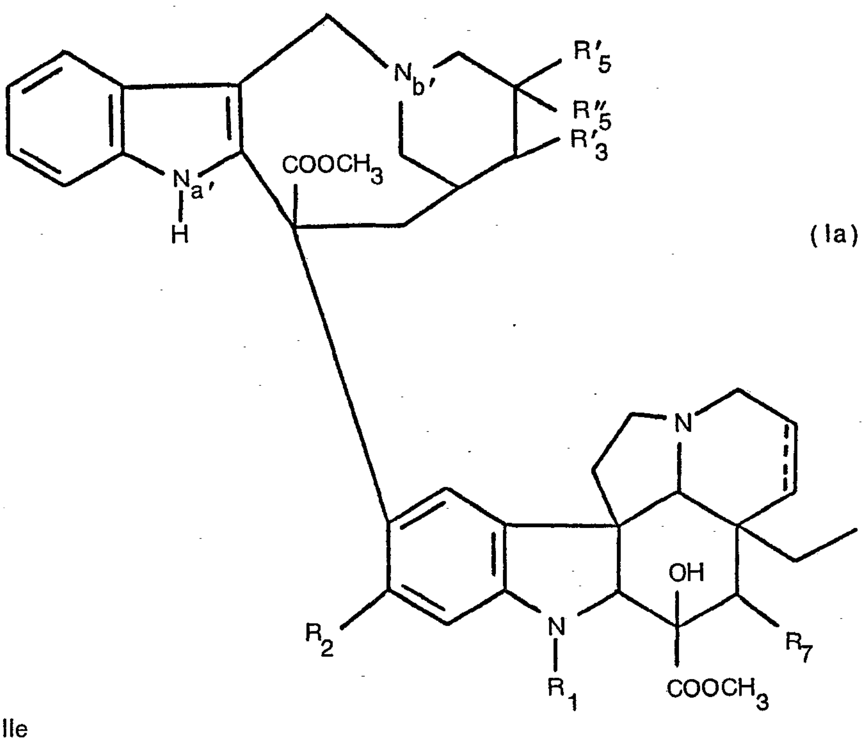

(Ia)

dans laquelle

$R'_3$ représente un atome d'hydrogène ou un radical hydroxy, et

$R'_5$ représente un atome d'hydrogène ou un radical hydroxy ou
$R'_3$ et $R'_5$ représentent ensemble un pont époxy ou une double liaison
$R''_5$ représente un atome d'hydrogène ou un radical éthyle,
$R_1$ représente un atome d'hydrogène, un radical alcoyle, formyle ou acyle;
$R_2$ représente un atome d'hydrogène, ou un radical méthoxy;
$R_7$ représente un radical alcanoyloxyle, la ligne pointillée représente une double liaison éventuelle; ainsi que les sels correspondants.

L'invention concerne en particulier:
de la nor-5′ anhydrovinblastine
nor-5′ anhydrovincristine
nor-5′ leurosine
chloro-12 nor-5′ anhydrovinblastine
nor-5′ $N_a$-Desméthyl Na-formyl leurosine.

La présent invention concerne également un procédé de préparation de composé de formule I, caractérisé en ce que

a) l'on fait réagir un composé de formule II

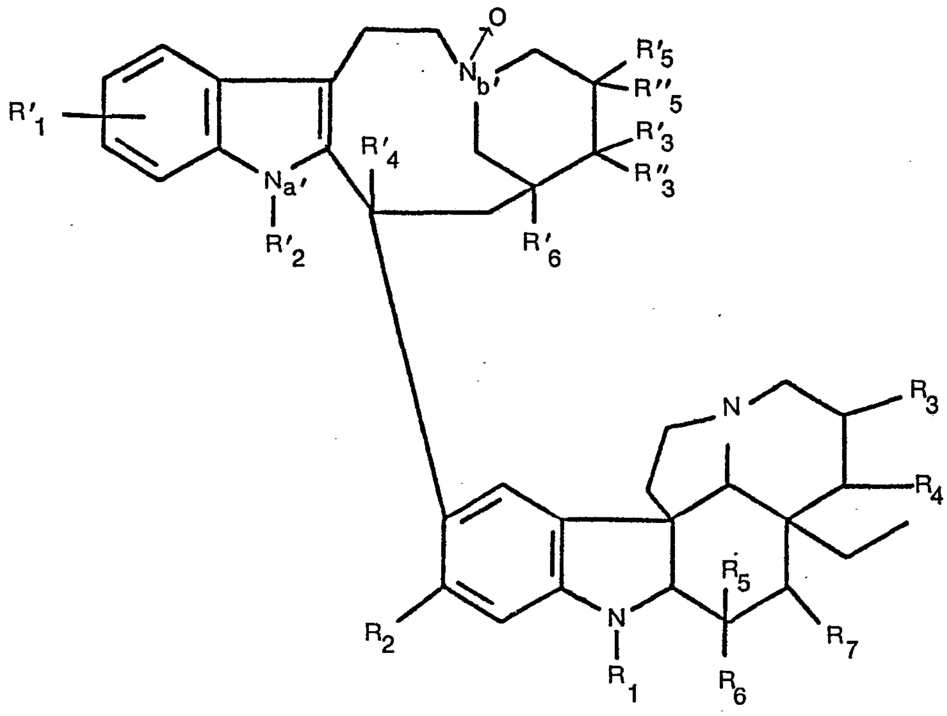

(II)

dans laquelle les substituants ont la signification donnée précédemment, en présence d'un réactif formateur d'ion immonium, et

b) en ce que l'on traite le produit obtenu par de l'eau, et sépare le composé de formule I.

Parmi les agents formateurs d'ion immonium, on utilise de préférence les halogénures, anhydrides ou sels d'acides organiques ou inorganiques en particulier d'acides carboxyliques halogénés (en particulier fluorés) ou non.

Les réactifs formateurs d'ion immonium sont par exemple l'anhydride d'acide acétique ou trifluoro-acétique.

L'étape a) est conduite de préférence dans un solvant organique anhydre tel qu'un solvant chloré, chlorure de méthylène, dichloroéthane ou chloroforme.

La réaction a) est conduite de préférence à une température comprise entre −5°C et +5°C par exemple 0°C.

L'étape b) est mise en oeuvre de préférence à l'aide d'un ou plusieurs solvants organiques non nucléophiles contenant de l'eau, comme solvant non nucléophile on utilise de préférence le tétrahydrofuranne ou le dioxanne. L'étape b) peut être conduite à température ambiante.

Lorsque l'on utilise un solvant différent dans l'étape a) et dans l'étape b), il est nécessaire de chasser le solvant du mélange réactionnel de l'étape a) avant la mise en oeuvre de l'étape b), on peut par exemple le distiller.

Il est possible également de conduire l'étape a) et l'étape b) dans un même solvant, dans ce cas l'étape a) est conduite dans le solvant anhydre et on rajoute de l'eau pour la mise en oeuvre de l'étape b).

La séparation de I peut être conduite par tout moyen connu de séparation, par exemple on peut

extraire le produit de formule I par un solvant chloré tel que le chloroforme, puis séparer le composé de formule I de la phase chloroformique par chromatographic préparative.

La séparation conduit en général à l'obtention d'un sous-produit qui, par réduction, donne un composé de formule III mentionné ci-dessous qui peut être recyclé.

Les composés de formule II sont connus (voir les brevets cités précédemment) ou peuvent être obtenus à partir de composés de formule III

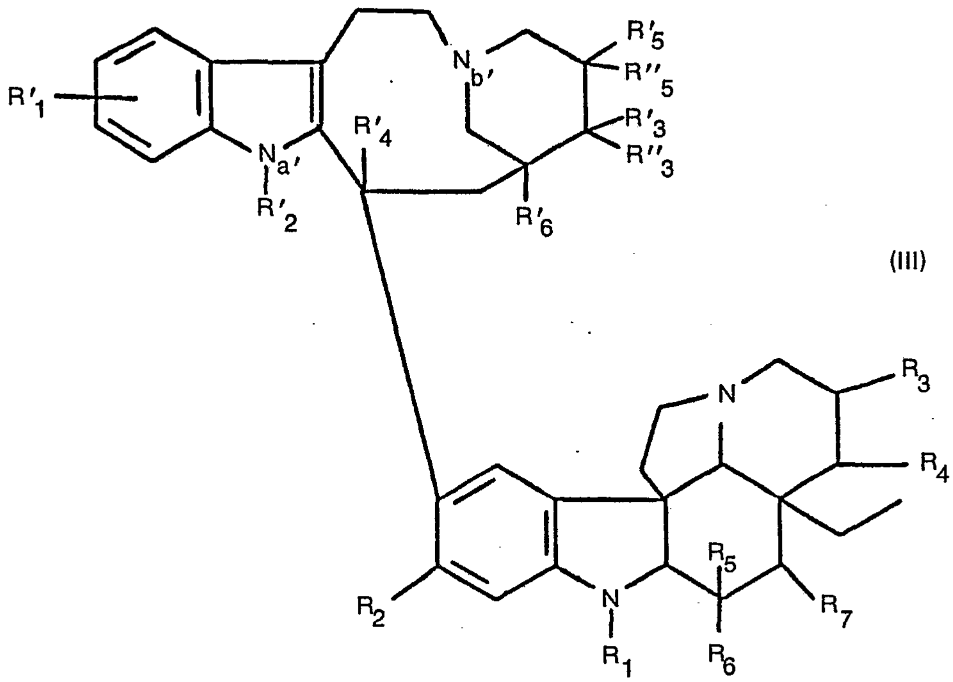

(III)

par des procédés connus dans ce domaine en particulier par oxydation par exemple à l'aide d'acides perbenzoïques dans un solvant notamment l'acide m-chloroperbenzoïque dans le chloroforme ou dans le chlorure de méthylène.

La présente invention concerne également un autre procédé de préparation des composés de formule I dans lequel on traite un composé de formule IV:

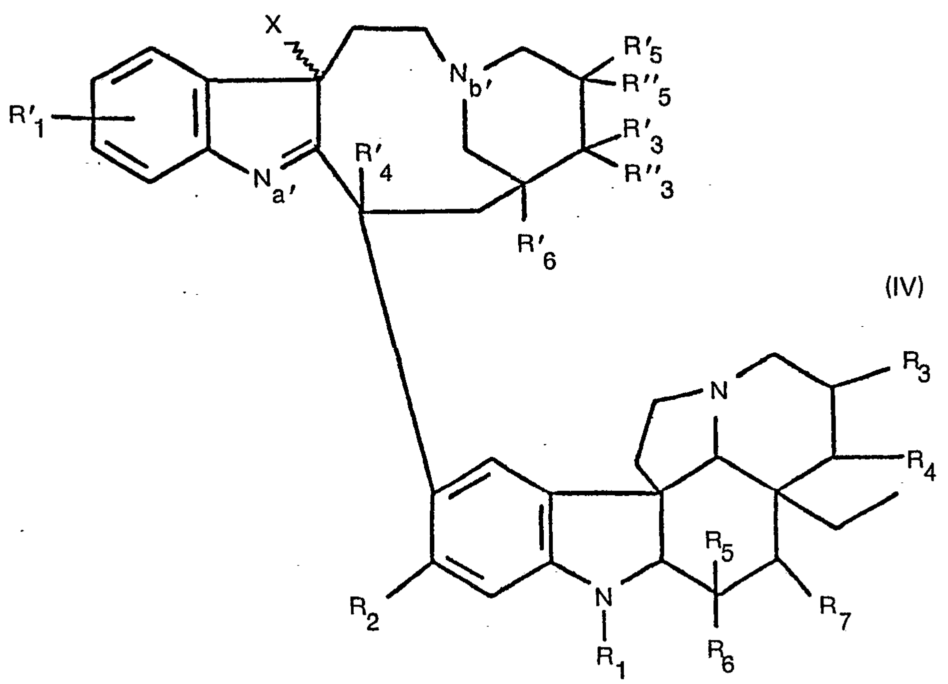

(IV)

dans laquelle les substituants ont la signification donnée précédemment et X représente un halogène, un radical hydroxy, hydroperoxy ou alcanoyloxy, par un mélange d'eau et d'au moins un solvant organique.

Les halogènes qui peuvent substituer les composés envisagés sont le fluor, le chlore, le brome et l'iode et en général il s'agira du chlore.

Le procédé selon la présent invention peut être mis en oeuvre sans catalyseur, mais la réaction peut être catalysée par des ions $Ag^+$ provenant de sels d'argent tels que le perchlorate, le tétrafluoroborate, le trifluoroacétate et l'acétate d'argent ou bien par un réactif acide, par exemple un acide inorganique tel que l'acide chlorhydrique par exemple.

Le solvant organique utilisé peut être quelconque et s'il est intéressant qu'il soit miscible à l'eau, il peut être non miscible sans que la réaction soit affectée par la présence de deux phases. Parmi les solvants utilisables, il faut citer plus particulièrement les éthers tels que le tétrahydrofuranne et le dioxanne et leurs mélanges.

Le rapport eau/solvant organique dans le mélange n'est pas critique mais on utilise, de préférence, un mélange comprise entre 30/70 et 70/30 en volume et de préférence 50/50 bien que l'on puisse descendre à des teneurs beaucoup plus faibles en $H_2O$ ou en solvant organique.

La température de réaction est de préférence comprise entre 0°C et 70°C.

La pression de réaction ne constitue pas un paramètre critique mais la réaction est conduite de préférence sous atmosphère inerte d'argon ou d'azote par exemple.

En fin de réaction, le composé de formule I peut être extrait par tout procédé connu mais il est particulièrement intéressant de l'extraire par un solvant organique chloré tel que le chloroforme.

La phase organique ainsi obtenue peut être évaporée sous vide et purifiée par exemple par chromatographie.

Les composés de formule générale IV peuvent être obtenus à partir de composés de formule III:

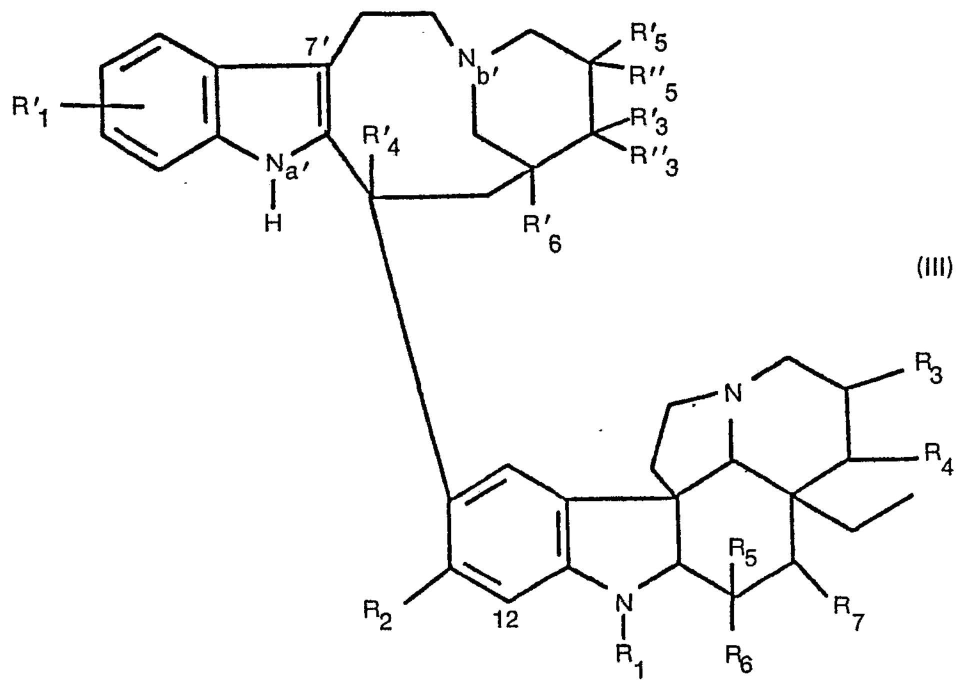

(III)

qui sont des composés connus ou qui peuvent être préparés notamment par les procédés décrits dans les brevets français n° 74 43221 et 77 11081.

Ainsi, pour préparer les composés de formule IV dans lesquels X est un halogène on effectue l'halogénation d'un composé de formule III avec un réactif générateur d'ion halogène positif, dans un solvant organique tel que le benzène, le chloroforme, le dichlorométhane, l'acétonitrile, le tétrahydrofuranne ou le dioxanne.

Les agents formateur d'ion halogène positif peuvent être les N-halogénobenzotriazoles comme le N-chlorobenzotriazole, un hypohalogénite comme l'hypochlorite de tertiobutyle ou l'hypochlorite de sodium, un N-halogénoamide tel que le N-chloroacétamide et le N-bromoacétamide ou un N-halogénoimide tel que le N-chlorosuccinimide ou le N-bromosuccinimide.

Cette réaction est conduite de préférence à une température comprise entre −10 et +20°C, en particulier à 0°C, sous atmosphère inerte bien que cette dernière condition ne soit pas indispensable.

Si l'on conduit la réaction avec des proportions de réactif voisines de la stoechiométrie, on obtient presqu'exclusivement les 7' halogéno-indolénines correspondantes. Mais, si on utilise le réactif générateur d'ion halogène positif en quantité double de la stoechiométrie, on obtient les dihalogéno 12,7' indolénines correspondantes qui par la réaction subséquente conduiront an 12 halogéno dirivé de formule I.

Si on le désire, les composés de formule IV peuvent être isolés du mélange réactionnel

d'halogénation par tout procédé connu, en particulier par extraction à l'aide d'un solvant chloré tel que le chloroforme, la phase ainsi obtenue peut être purifiée, par exemple, par évaporation sous vide et chromatographie. Evidemment, compte tenu de ce qui précède, si l'on désire isoler les composés de formule IV il est nécessaire d'opérer en milieu anhydre. Il n'est toutefois pas nécessaire en général d'isoler le composé de formule IV qui peut être utilisé directement dans son milieu réactionnel pour la préparation du composé de formule I.

Il est également possible de préparer directement le composé de formule I, en combinant la formation du composé de formule IV et la formation du composé de formule I, en faisant réagir sur le composé de formule III le réactif générateur d'ion halogène positif dans un solvant organique contenant de l'eau, le composé de formule IV n'apparaît alors que de façon transitoire.

Dans ce dernier cas, on peut également prévoir d'ajouter au mélange des ions $Ag^+$ ou un réactif acide afin d'accélérer la réaction de formation du composé de formule I.

Dans le cas de composés de formule II dans lesquels X représente le radical hydroperoxyde, ces composés peuvent être préparés par action de l'oxygène sur les composés de formule III en présence par exemple d'un sel métallique.

Les composés de formule IV dans lesquels X représente le radical hydroxy peuvent être préparés à partir des composés dans lesquels X représente un radical hydroperoxyde par réduction à l'aide d'un trialcoyle ou triaryle phosphite tel que le triéthyle phosphite.

Dans le cas de composés dans lesquels X représente un radical alcanoyloxy, ces composés peuvent être préparés par action d'un tétracarboxylate de plomb tel que le tétracétate de plomb sur les composés de formule III.

D'autres caractéristiques de mise en oeuvre de la présente invention pourront être déduites des exemples suivants:

### Exemple (Schéma I) 1

a) *Préparation du Nb' oxyde de $\Delta^{15'}$ deshydroxy-20' vincaleucoblastine (II')*

A une solution de $\Delta^{15'}$ deshydroxy-20' vincaleucoblastine (III') (748 mg; 0,94 mmole) dans 10 ml de chlorure de méthylène à 0°C sous agitation et sous argon, on ajoute en une seule fois 180 mg (1,04 mmole) d'acide méta chloro-perbenzoïque. On laisse en contact 30 mm à 0°C. Puis on reprend le milieu réactionnel par 100 ml de chloroforme et on lave la phase organique 4 fois avec 3 ml d'une solution aqueous de bicarbonate de sodium à 40 g/l. La phase organique est ensuite lavée à neutralité par de l'eau saturée de chlorure de sodium (3 fois 10 ml); séchée par du sulfate de sodium, filtrée et évaporée sous vide pour fournir 712 mg de Nb' oxyde de $\Delta^{15'}$ deshydroxy-20' vincaleucoblastine (Rdt: 93%).

U.V. ($\lambda$ nm, $\varepsilon$): 213, 268, 289, 293, 310. RMN¹H (400 MHZ; $\delta$=OPPM.TMS; J: Hz) 9,50 (s, 1H), $C_{16}$—OH; 8,19 (s, 1H) Na'—H; 7,70 (d, 1H, J$\simeq$7,5) $C_{9'}$ H ou $C_{12'}$ H; 7,19 et 7,14 $C_{10'}$ H et $C_{11'}$ H; 7,07 (d, J$\simeq$7,5) $C_{12'}$ H ou $C_9$ H; 6,43 (s, 1H) $C_9$ H; 6,11 (s, 1H) $C_{12}$ H; 5,84 (dd, 1H, $J_{14,15}\simeq$10Hz et $J_{3,14\simeq 4,5}$ $C_{14}$ H; 5,44 (1H) $C_{15'}$ H, 5,41 (s, 1H) $C_{17}$—H; 5,27 (d, 1H, $J_{14,15}\simeq$10Hz)$C_{15}$ H; 4,51 (m, 2H) 4,32 (d, 1H) et 3,98 (d, 1H: Nb'CH; 3,84 (s, 3H), 3,78 (s, 3H) et 3,69 (s, 3H) $C_{11}OCH_3$, $C_{16}CO_2CH_3$ et $C_{16'}$ $CO_2CH_3$ 2,62 (s, 3H) $NaCH_3$; 2,09 (2, 3H) $OCOCH_3$; 1,06 (t, 3H, $J_{18',19'}$: 7Hz) $C_{18'}$ H et 0,64 (t, 3H, $J_{18,19\simeq 7}C_{18}$H — S.M.: 808, 806, 792 (M—16), 777, 871, 733, 669, 633, 631, 612, 611, 610, 510, 469, 282, 222, 200, 193, 144, 136, 135, 122, 121 (pic de base), 108, 107.

b) *Préparation de la nor-6' $\Delta^{15'}$ deshydroxy-20' vincaleucoblastine (I')*

A une solution du $N_{b'}$ oxyde précédent (345 mg; 0,43 mmole) dans 2 ml de chlorure de méthylène sec à 0°C sous agitation et sous argon, on ajoute 276 $\mu$l d'anhydride trifluoroacétique (1,89 mmole). On laisse en contact 2 h à 0°C. Le milieu réactionnel est évaporé à sec à la pompe électrique et repris par 8 ml de tétrahydrofuranne et 30 ml d'eau, agité à température ambiante pendant 1 heure, repris par 50 ml de chloroforme et lavé avec une solution aqueuse saturée de chlorure de sodium. La phase organique est ensuite séchée et évaporée. Le produit brut ainsi obtenu est purifié par chromatographie sur plaque épaisse de silice (éluant $CHCl_3$.MeOH 90:10). Le produit le moins polaire (90 mg; 27%) correspond au composé I'.

$[\alpha_D]$ = +52,4 (c=3,24) $CHCl_3$ U.V. ($\lambda$nm, $\varepsilon$): 215 (36.700), 268 (11.000), 282 (9,500), 293 (7.600), 310 (4.400). D.C. ($\lambda$nm, $\Delta\varepsilon$): 312 (+3,4), 305 (+3,2), 258 (+13,3), 230 (+31,6) 210 (−44,3). RMN¹H (400 MHZ; $\delta$ OPPM.TMS; J:Hz); 8,50 (s, 1H) OH ou NH; 7,77 (d, 1H, J=8) NH ou aromatique; 7,15 (m, 4H) aromatique; 6,34 (s, 1H) $C_9$H; 6,08 (S, 1H) $C_{12}$—H; 5,83 (dd, 1H, J=3,5 et 9,5) $C_{14}$H; 5,76 (m, 1H) $C_{15'}$—H; 5,37 (s, 1H) $C_{17}$H; 5,25 (d, 1H, J=9,5) $C_{15}$H; 4,58 (m, 1H) et 4,41 (d, 1H) $C_{6'}$H et $C_{6'}$H'; 3,83 (s, 3H); 3,13 (s, 3H); 3,68 (s, 4H) $C_{16}CO_2CH_3$, $C_{11}OCH_3$, $C_{16'}CO_2CH_3$ et $C_{21}$H; 2,69 (s, 3H) Na—$CH_3$; 2,57 (s, 1H) $C_2$H; 2,06 (s, 3H) $OCOCH_3$; 1,07 (t, 3H, J=7); 0,70 (t, 3H, J=7) $C_{18'}$H et $C_{18}$ H. S.M.: 794, 792, 748, 656, 598, 522, 480, 450, 436, 331, 282, 240, 222, 210, 165, 152, 144, 136, 135, 122. I.R. ($\nu$ $cm^{-1}$): 1740, 3420.

Le produit le plus polaire (275 mg) repris par du MeOH (3 ml); réduit par un excès de borohydrure

de sodium, extrait et purifié comme plus haut fournit 200 mg de $\Delta^{15'}$ deshydroxy-20' vincaleucoblastine (III') qui peut être recyclé.

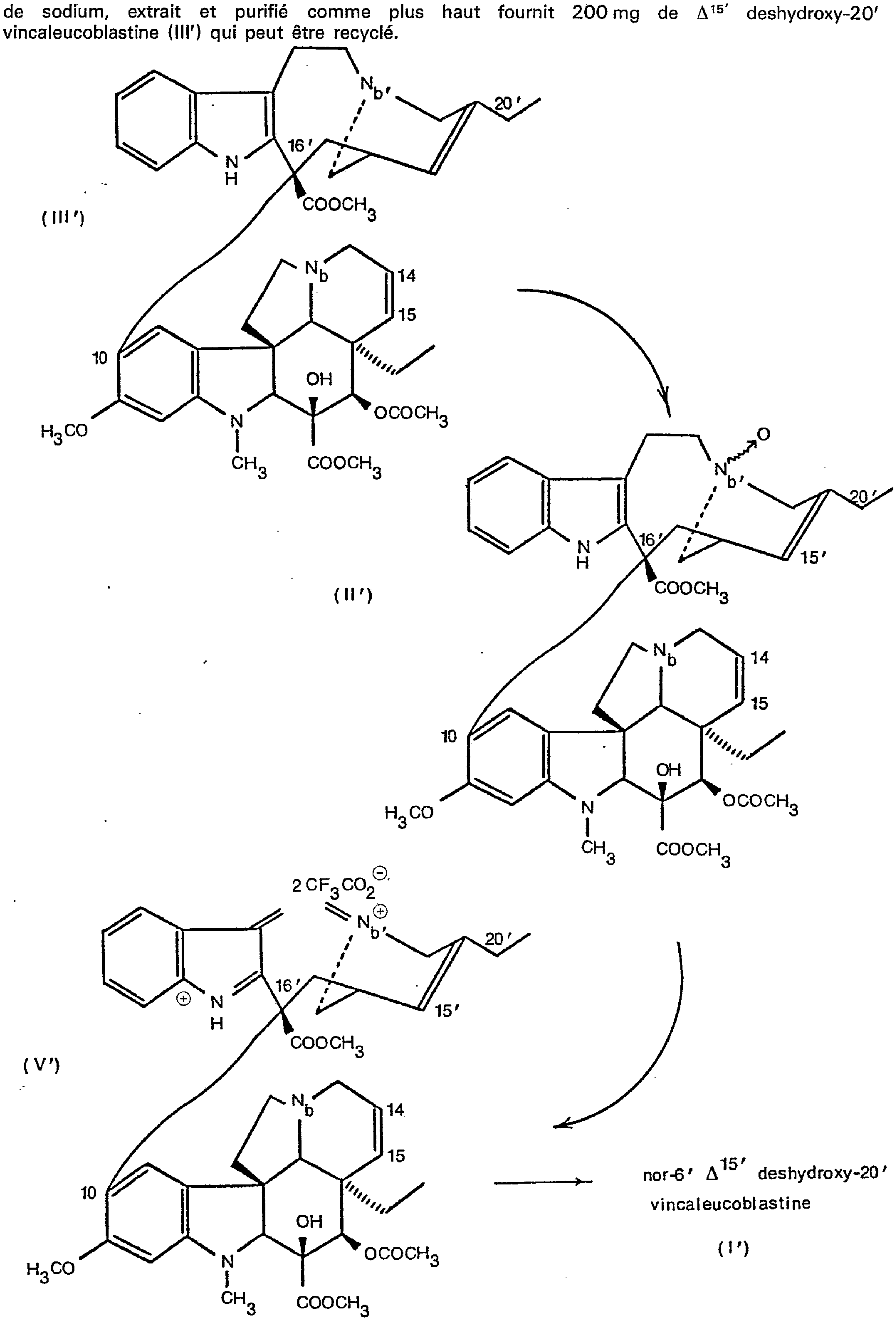

(III')

(II')

(V')

nor-6' $\Delta^{15'}$ deshydroxy-20' vincaleucoblastine

(I')

SCHEMA I

Exemple 2

a) *Préparation de la chloro-7′ indolénine de l'anhydrovinblastine (formule IV′)*

A une solution de 330 mg d'anhydrovinblastine (formule III′) (0,42 mM) dans 10 cm³ de chlorure de méthylène anhydre, maintenue sous atmosphère d'argon, on ajoute à 0°C et sous agitation une solution de 76 mg de N-chlorobenzotriazole (0,49 mM) dans 20 cm³ du même solvant. Après 40 minutes, le solvant est éliminé sous pression réduite à basse température (<15°C). La chloro-7′ indolénine de l'anhydrovinblastine (72 mg, rendement 21%) est séparée, par chromatographie sur plaque de silice (éluant: $CHCl_3$—MeOH: 95—5), de l'anhydrovinblastine de départ (140 mg, 42%) qui peut être recyclée et des autres sous produits.

La chloro-7′ indolénine présente les caractéristiques suivantes:

IR ($CHCl_3$): 3460, 2920, 1745, 1620, 1505, 1465, 1435 $cm^{-1}$.

UV (EtOH) $\lambda$ max ($\varepsilon$): 215 (61000); 255 (21000); 313 nm (12250)

($EtOH+H^+$) $\lambda$ max: 216, 260, 302 nm: indoléninedihydroindole.

DC (EtOH) $\lambda$ nm ($\Delta\varepsilon$): 215 (—70): 230 (+35,8): 258 (—2,0); 285 (+6,9): 311 (—4,1); 345 (+15,1).

SM pics à m/e: 792, 748, 703, 691, 612, 598 (100%) 538, 522, 480, 450, 331, 329, 282, 222, 200, 165, 152, 144, 136, 135, 122, 121, 107.

RMN du 1H ($CDCl_3$, $\delta$=0 ppm TMS, 240 MHz): 7,58 (s, large, 1H, attribué à $C_9$—H): 7,3—7,1 (aromatiques); 5,91 (s, 1H, $C_{12}$—H); 5,80 (dd, 1H, $J_{14,15}$=9 et $J_{3,14}$=3 Hz, $C_{14}$—H); 5,43 (s, 1H, $C_{17}$—H); 5,24 (m, 1H, $C_{15'}$—H); 5,05 (d, 1H, J~9 Hz, $C_{15}$—H); 3,78—3,71 et 3,52 (3s, 9H, $C_{11}$—$OCH_3$, $C_{16}$—$CO_2$ $CH_3$ et $C_{16'}$—$CO_2CH_3$); 2,62 (s, 3H, $N_a$—$CH_3$); 2,03 (s, 3H, $COCH_3$); 1,00 (t, 3H, J~7,5 Hz, attribué à $C_{18'}$—H) et —0,28 (attribué à $C_{18}$—H).

b) *Préparation de la nor-5′ anhydrovinblastine (formule I′) à partir de la chloro-7′ indolénine de l'anhydrovinblastine*

Une solution de 9,0 mg d'$AgBF_4$ (0.046 mM) dans 7 cm³ d'un mélange THF—$H_2O$ 50—50 v/v est ajoutée à 33 mg de la chloro-7′ indolénine obtenue à l'étape a) maintenue sous atmosphère d'argon. Le mélange est agité pendant 4 h à 50°C puis refroidi, concentré sous pression réduite à 25—30°C, dilué par 10 cm³ d'une solution aqueuse de $Na_2CO_3$ à 10% et extrait par du chloroforme. Les phases organiques sont séchées sur $Na_2SO_4$ et filtrées. L'élimination du solvant sous pression réduite permet d'obtenir quantitativement 30 mg de nor-5′ anhydrovinblastine identique à un échantillon préparé selon le procédé décrit dans l'exemple 1.

Le schéma II décrit la préparation de ce composé par le procédé de l'invention.

Exemple 3

a) *Préparation de la dichloro-12,7′ indolénine de l'anhydrovinblastine*

A une solution de 50 mg d'anhydrovinblastine (0,063 mM) dans 1,7 cm³ de chlorure de méthylène anhydre maintenue sous argon à 0°C on ajoute une solution de 10,7 mg de N-chlorobenzotriazole (0,07 mM) dans 1,5 cm³ de chlorure de méthylène, puis 11,3 mg du même réaction. Après 45 minutes d'agitation le milieu réactionnel est traité comme décrit dans l'exemple 1a). La dichloro-12,7′ indolénine de l'anhydrovinblastine, après purification par chromatographie sur plaque de silice (éluant: $CHCl_3$—MeOH 93—7), est obtenue avec un rendement de l'ordre de 45% et présente les caractéristiques suivantes:

IR ($CHCl_3$): 3480, 2920, 1750, 1615, 1465 $cm^{-1}$.

UV (EtOH) $\lambda$ max ($\varepsilon$): 223 (35500); 255 (10700); 310 nm (7200)

($EtOH+H^+$) $\lambda$ max: 226, 262, 305 nm: indoléninedihydroindole.

DC (EtOH) $\lambda$ max ($\Delta\varepsilon$): 233 (+11,5); 259 (—2,2); 300 (+4,5) 325 (+5,1).

Sm pics à m/e: 826, 782, 752, 737, 632, 591, 572, 556, 514, 484, 365, 363, 282, 222, 182, 167, 152, 144, 136, 135 (100%), 122, 121, 120, 107, 106.

RMN du 1H ($CDCl_3$): 7,53 (1H, attribué à $C_9$—H): 7,34 et 7,25 (aromatiques); 5,77 (dd, 1H, $J_{14,15}$=10 et $J_{3,14}$=4 Hz, $C_{14}$—H); 5,41 (s, 1H, $C_{17}$—H); 5,29 (m, 1H, $C_{15'}$—H); 5,08 (d, 1H, $J_{14,15}$=10 Hz, $C_{15}$H); 3,94, 3,78 et 3,53 (3s, 9H, $C_{11}$—$OCH_3$, $C_{16}$—$CO_2CH_3$ et $C_{16'}$—$CO_2CH_3$); 2,86 (s, 3H, $N_aCH_3$); 2,03 (s, 3H, $COCH_3$); 1,01 (t, 3H, J=7 Hz, attribué à $C_{18'}$—H) et —0,17 ppm (attribué à $C_{18}$—H).

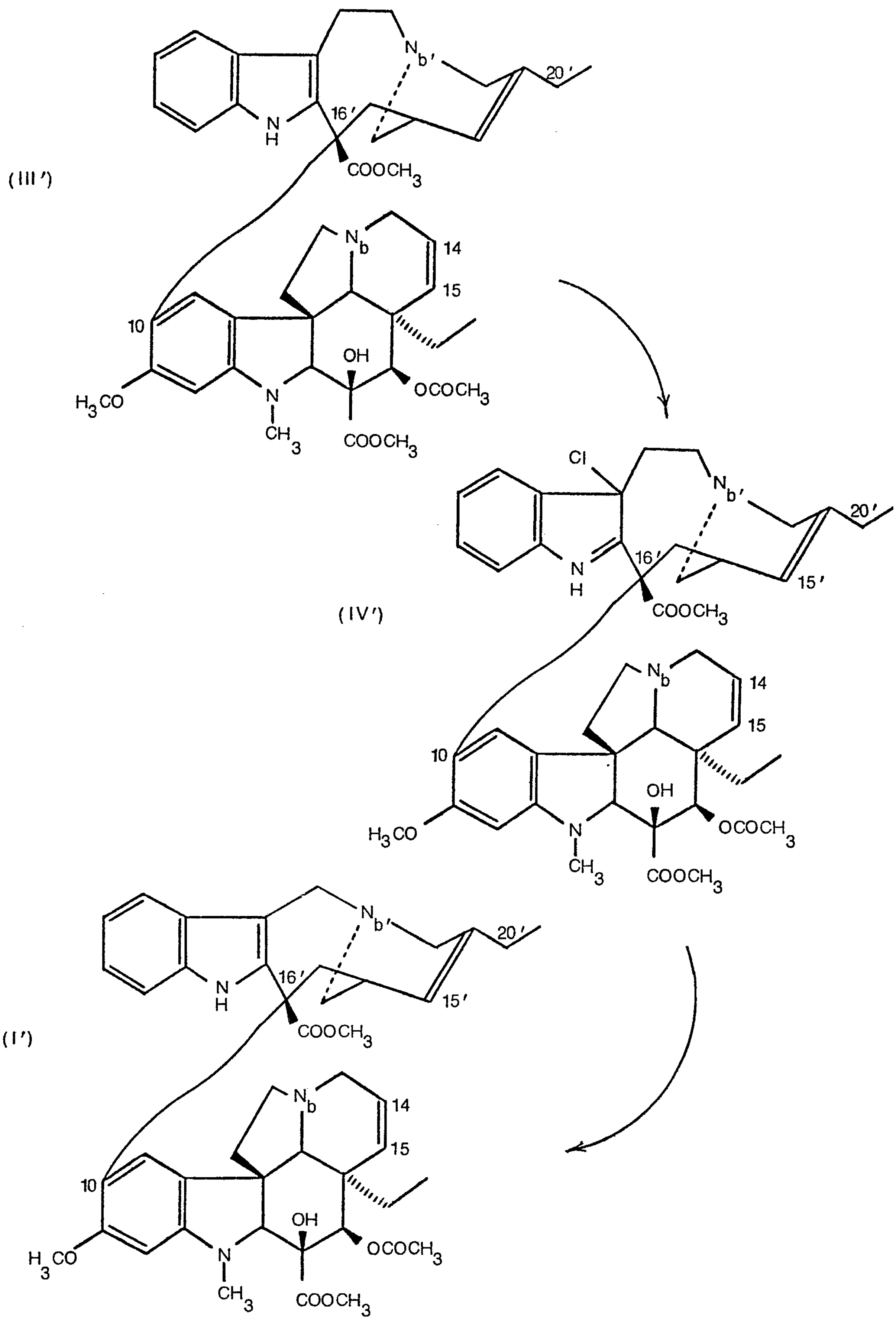

(III')

(IV')

(I')

SCHEMA II

b) *Préparation de la chloro-12 nor-5' anhydrovinblastine*

A une solution de 22 mg de chloro-12 chloro-7' indolénine obtenue à l'étape a) (2,5.10$^{-5}$ M) dans 1,2 cm$^3$ d'un mélange THF—$H_2O$ 50—50 (V/V) on ajoute sous argon et à 0°C une solution de 6 mg d'$AgBF_4$ (3.10$^{-5}$ M) dans 2,8 cm$^3$ du même mélange. Le milieu réactionnel est agité à 40—45°C pendant 17 heures puis traité comme décrit dans l'exemple 1b) pour obtenir 19,6 mg de chloro-12 nor-5' anhydrovinblastine (rendement 95%) ayant les caractéristiques suivantes:

$[\alpha]_D^{15}$ + 22° ($CHCl_3$, C=0,59)

IR ($CHCl_3$): 3480, 3460, 2920, 1750, 1610, 1462 cm$^{-1}$

UV (EtOH) $\lambda$ max ($\varepsilon$): 221 (46000); 272 (17000); 293 (10500) et 308 nm (4000).

DC (EtOH) $\lambda$ max ($\Delta\varepsilon$): 216 (−46,2); 231 (+29,1); 245 (+11,5); 262 (+25,7); 293 (−1,1); 306 (+3,4); 320 (+3,4)

SM pic à m/e: 828, 826, 782, 768, 725, 646, 632, 565, 282, 222, 152, 144, 136 (100%), 135, 123, 122, 121, 107.

RMN du 1H ($CDCl_3$): 8,47 (s, 1H, $N_{a'}$—H); 7,69 (1H, arom.indolique); 7,14 (3H, arom.indoliques); 6,36 (s, 1H, $C_9$—H); 5,89 (dd, 1H, $J_{14,15}$=10 et $J_{3,14}$~4 Hz, $C_{14}$—H); 5,70 (1H, $C_{15'}$—H); 5,29 (s, 1H, $C_{17}$—H); 5,27 (1H, $C_{15}$—H); 4,28 (dd, 2H, $J_{6a,6b}$=14 Hz, $C_{6'}$—H); 3,95, 3,78 et 3,75 (3s, 9H, $C_{11}$—$OCH_3$, $C_{16}$—$CO_2CH_3$ et $C_{16'}$—$CO_2CH_3$); 3,70 (s attribué à $C_2$—H); 2,97 (s, 3H, $N_a$—$CH_3$); 2,09 (s, 3H, $COCH_3$); 1,06 (t, 3H, $J_{18',19'}$=6,5 Hz, $C_{18'}$—H) et 0,70 ppm (t, 3H, $J_{18,19}$=6 Hz, $C_{18}$—H).

## Exemple 4

a) *Préparation de la chloro-7' indolénine de l'anhydrovincristine*

A une solution de 120 mg d'anhydrovincristine (0,15 mM) préparée comme décrit (J.P. Kutney, J. Balsevitch, T. Honda, PH. Liao, HPM. Thillied et BR. Worth, Can. J. of Chem. (1978), 56, p. 2560) dans 12 cm$^3$ de chlorure de méthylène anhydre maintenue sous argon à 0°C, on ajoute, sous agitation, 29 mg de N-chlorobenzotriazole (0,19 mM). Après 2 heures 30 minutes d'agitation, le milieu réactionnel est évaporé à sec sous pression réduite à une température inférieur à 20°C. Par chromatogrphie sur plaque de silice (éluant: AcOEt—MeOH 90—10) on sépare l'anhydrovincristine qui n'a pas réagi (20 mg, 17%) de la chloro-7' indolénine de l'anhydrovincristine (87 mg, rendement 70%) qui présente les caractéristiques suivantes:

IR: 3000, 1760, 1680, 1615, 1600 cm$^{-1}$.

UV (EtOH) $\lambda$ max: 222, 256, 310 nm

(EtOH+H$^+$) $\lambda$ max: 225, 254, 296 nm.

DC (EtOH) $\lambda$ nm: 235(+); 300(−); 320(+).

(EtOH+H$^+$) $\lambda$ nm: 225(+); 260(+); 305(+).

SM: 806, 804, 773, 762, 645, 612, 610, 552, 536, 494, 466, 401, 366, 358, 329, 282, 136 (100%), 135, 122, 121.

b) *Nor-5' anhydrovincristine*

A une solution de 60 mg de chloro-7' indolénine de l'anhydrovincristine (0,07 mM) dans 3 cm$^3$ d'un mélange THF—$H_2O$ 50—50 (V/V), on ajoute à température ordinaire et sous agitation 20 mg d'$AgBF_4$ (0,1 mM). Le mélange est agité à 45°C pendant 2 heures 30 minutes puis extrait par du chloroforme en présence d'une solution aqueuse de $Na_2CO_3$ à 10%. Après lavage à l'eau, séchage sur $Na_2SO_4$ et évaporation du solvant sous pression réduite on isole 51 mg (rendement 90%) de nor-5' anhydrovincristine ayant les caractéristiques suivantes:

IR ($CHCl_3$): 3400, 2950, 1745, 1680 cm$^{-1}$

UV (EtOH): 215, 259, 282, 292 nm

DC (EtOH) $\lambda$ nm: 205(−); 220(+); 250(+); 295 nm(+)

SM pics à m/e: 806, 794, 792, 776, 762, 645, 630, 610, 599, 587, 536, 508, 494, 466, 152, 136 (100%), 135, 122, 121.

RMN 1H ($CDCl_3$): 8,76 (s, 1H, OH); 8,48 (s large, 1H, $N_{a'}$—H); 8,18 (0,5H) et 7,73 (1,5H); $N_a$CHO + aromatique; 7,18 3H, aromatiques); 6,80 (s, 1H, $C_9$—H); 6,71 (2s, 1H, $C_{12}$—H); 5,91 (m, 1H, $C_{14}$—H); 5,70 (m, 1H, $C_{15'}$—H); 5,40 (d, 1H, $J_{14,15}$=10 Hz, $C_{15-H}$); 5,20 (2s, 1H, $C_{17}$—H); 4,75 et 4,50 (2s, 1H, $C_2$—H); 3,91, 3,75 et 3,71 (3s, 9H, $C_{11}$—$OCH_3$, $C_{16}$—$CO_2CH_3$ et $C_{16'}$—$CO_2CH_3$); 2,09 (2s, 3H, $COCH_3$); 1,08 (t, 3H, J=7 Hz) et 0,71 ppm (3H), $C_{18'}$—H et $C_{18}$—H).

## Exemple 5

a) *Chloro-7' indolénine de la leurosine*

A une solution de 100 mg de leurosine (0,124 mM) dans 10 cm$^3$ de chlorure de méthylène anhydre, maintenue, sous argon à 0°C, on ajoute 23 mg de N-chlorobenzotriazole (0,15 mM). Après 1 heure 45 minutes d'agitation à 0°C et évaporation du solvant sous pression réduite à une température inférieure à 20°C, les produits sont séparés par chromatographie sur silice (éluant: AcOEt—EtOH 3—1). On isole ainsi 62 mg (rendement 57%) de chloro-7' indolénine de la leurosine et 30 mg de leurosine qui peut être recyclée.

La chloro-7′ indolénine de la leurosine présente les caractéristiques suivantes:
IR ($CHCl_3$): 3000, 1750 $cm^{-1}$
UV (EtOH) λ max: 220, 248, 300 nm.
DC (EtOH) λnm: 245(+), 295(−), 325(+)
($EtOH+H^+$) λnm: 210(−), 225(+), 270(−), 295(+)
SM pics à m/e: 844, 843, 842, 841, 807, 684, 682, 670, 648, 602, 494, 352 (100%), 310, 308, 283, 154, 135, 122, 121.

b) *Nor-5′ leurosine*

A une solution de 40 mg de chloro-7′ indolénine de leurosine (40 mg, $4{,}75.10^{-5}$ M) dans 4 $cm^3$ d'un mélange de THF—$H_2O$ 50—50 (V/V), on ajoute 10 mg d'$AgBF_4$ et on agite le mélange obtenu à 50°C pendant 3 heures. Après extraction comme décrit dans l'exemple 3b) on obtient la nor-5′ leurosine (35 mg, rendement 93%), identique à un échantillon préparé par un procédé similaire au procédé décrit dans l'exemple 1, ayant les caractéristiques suivantes:
IR ($CHCl_3$) 3350, 2950, 1750 $cm^{-1}$
$[\alpha]_D^{20°} = 32°$ (C=0,5, $CHCl_3$)
UV (EtOH) λ max: 217, 270, 285 (ep.), 293 (ep.), 311 nm
DC (EtOH) λ nm: 215(−), 220(+), 255(+), 280(−), 310(+).
SM pics à m/e: 810, 796, 794, 761, 750, 656, 649, 637, 633, 598 (100%), 538, 522, 496, 480, 469, 450, 449, 448, 369, 367, 340, 331, 329, 282, 240, 238, 222, 210, 208, 188, 174, 165, 154, 152, 135, 122, 121, 107.
RMN du 1H ($CDCl_3$): 8,31 (s, 1H, NaH); 7,55 (1H, aromatique); 7,06 (3H aromatiques); 6,30 (s, 1H, $C_9$—H); 6,00 (s, 1H, $C_{12}$—H); 5,76 (dd, 1H, $J_{14'15}$=10 et $J_{3,14}$=4 Hz, $C_{14}$—H); 5,31 (s, 1H, $C_{17}$—H); 5,23 (d, 1H, $J_{14,15}$=10, $C_{15}$—H); 4,30 et 4,11 (2d, $J_{6'a,6'b}$=13 Hz, $C_{6'}$—H); 3,76, 3,73 et 3,66 (3s, 9H, $C_{11}$—$OCH_3$, $C_{16}$—$CO_2CH_3$ et $C_{16'}$—$CO_2CH_3$); 2,66 (s, 3H, $N_a$—$CH_3$); 2,05 (s, 3H, $COCH_3$); 1,05 et 0,66 ppm (2t, 6H, J=7 Hz, $C_{18}$—H et $C_{18'}$—H).

Exemple 6

*Préparation de la chloro-7′ indolenine de la $N_a$- desméthyl $N_a$-formyl leurosine:*

A une solution de 60 mg de $N_a$-desméthyl $N_a$-formyl leurosine ou leuroformine (0,072 mM) dans 6 $cm^3$ de chlorure de méthylène anhydre maintenue sous argon à 0°C, on ajoute, sous agitation, 14 mg de N-chloro benzotriazole (0,092 mM). Après 2 heures d'agitation, le milieu réactionnel est évaporé à sec sous pression réduite à une température inférieure à 20°C; on ajoute 1,5 $cm^3$ de méthanol, 10 $cm^3$ d'une solution de carbonate de sodium aqueux (40%) et on extrait par du benzène. Après lavage à l'eau, séchage sur $Na_2SO_4$, évaporation du solvant sous pression réduite et purification sur chromatographie sur plaque de silice éluant $CHCl_3$—$CH_3OH$ 95—5 saturé de $NH_3$) on obtient la chloro-7′ indolénine de la $N_a$-desméthyl $N_a$-formyl leurosine (52 mg, rendement 85%) qui présente les caractéristiques suivantes:
IR ($CHCl_3$): 3000, 1760, 1690, 1600 $cm^{-1}$.
UV (EtOH) λ max: 226, 256, 310 nm.
(EtOH + $H^+$) λ max: 225, 254, 298 nm.
DC (EtOH) λ nm: 205(−); 235(+); 258(+); 295(−); 325(+).
(EtOH + $H^+$) λ nm: 230(+); 255(+); 295(+).
SM: 836, 822, 616, 365, 282, 149, 133, 122 (100%), 121.

*Nor-5′ $N_a$-Desméthyl $N_a$-formyl leurosine:*

A une solution de 50 mg de chloro-7′ indolénine de $N_a$-desméthyl $N_a$-formyl leurosine (0,07 mM) dans 4 $cm^3$ d'un mélange THF—$H_2O$ 50—50 (v/v), on ajoute à température ordinaire et sous agitation 20 mg d'$AgBF_4$ (0,1 mM). Le mélange est agité à 20°C pendant 16 h puis extrait par de l'éther en présence de $Na_2CO_3$ à 10%. Après séchage sur $Na_2SO_4$, évaporation du solvant sous pression réduite et purification par chromatographie sur plaque de silice (éluant $CHCl_3$—$CH_3OH$ 95—5 cuve saturée de $NH_3$), on isole 20 mg de nor-5′ $N_a$ desméthyl-$N_a$ formyl leurosine (Rdt 40%) ayant les caractéristiques suivantes:
IR ($CHCl_3$): 3400, 2950, 1750, 1680 $cm^{-1}$.
UV (EtOH) λ max: 222, 260, 286, 295 nm.
DC (EtOH) λ: 205(−); 225(+); 255(+); 298(+).
SM pics à m/e: 824, 822, 764, 612, 610, 584, 494, 282, 154, 152, 144, 136 (100%), 122, 121.
RMN$^1$H $CDCl_3$: 8,70 (s, 1H, OH), 8,41 (s large, 1H, $N_{a'}$—H); 8,12 (0,5 H) et 7,70 (0,5 H); NaCHO; 7,62 (d, J=7 Hz, 1H aromatique); 7,12 (m, 3H, aromatiques); 6,76 (s, 1H, $C_9$—H); 6,64 (2s, 1H, $C_{12}$—H); 5,86 (dd, $J_{14,15}$=10 Hz, $J_{3,14}$=3 Hz, 1H, $C_{14}$—H); 5,33 (d, $J_{14,15}$=10, 1H, $C_{15}$—H), 5,16 (2s, 1H, $C_{17}$—H), 4,70 et 4,45 (2s, 1H, $C_2$—H), 4,32 (d, $J_{AB}$=12 Hz, 1H, $C_{6'}$—H); 4,21 (d, $J_{AB}$=12 Hz, 1H, $C_{6'}$—H′); 3,92; 3,76 et 3,68 (3s, 9H) $C_{11}OCH_3$, $C_{16}CO_2CH_3$,$C_{16'}CO_2CH_3$); 2,05 (2s, 3H, $OCOCH_3$); 1,07 (t, $J_{18,19}$=7 Hz, 3H) et 0,70 (t, $J_{18,19}$=7 Hz, 3H) $C_{18}$—H et $C_{18'}$—H.

Les composés de formule I selon la présente invention présentent des propriétés anti-tumorales et peuvent donc être utilisés dans le traitement de diverses affections tumorales telles que des leucémies.

La présente invention concerne donc également à titre de médicaments nouveaux les composés de formule I ainsi que les compositions pharmaceutiques les contenant.

On donne ci-après les résultats des études pharmacologiques in-vitro et in vivo conduites sur les composés des exemples précédents.

1) *Inhibition de la polymérisation de la tubuline*

Le récepteur des alcaloïdes indoliques antitumoraux de type vinblastine est la tubuline.

Elle est facilement extraite du cerveau de porc où elle représente 10% des protéines solubles.

La polymérisation de la tubuline en microtubules peut être facilement suivie au moyen d'un spectrophomètre UV en observant à 350 nm. La vitesse maximale de polymérisation est ainsi déterminée; celle-ci est diminuée par l'addition d'inhibiteurs du type de la vinblastine et une concentration, $I_{50}$, diminuant de moitié la vitesse est obtenue pour chaque substance essayée. Un deuxième effet de ces produits est pris en considération: à des doses plus élevées, après inhibition totale de la polymérisation, une spiralisation de la tubuline est observée (vérifié par microsopie électronique) et une nouvelle concentration, $S_{50}$, correspondant à l'apparition de 50% du phénomène est déterminée.

Les résultats de chaque produit sont toujours comparés à ceux de la vinblastine utilisée comme référence.

TABLEAU I

Les résultats des tests d'interaction avec la tubuline effectués sur les tartrates sont résumés dans le tableau suivant (concentration en tubuline ∼2 mg/ml):

| Composé | $I_{50}/I_{50}$ vinblastine (a) | $S_{50}/S_{50}$ vinblastine (b) |
|---|---|---|
| Nor-5' anhydrovinblastine | 0,7 | 0,8–1,0 |
| Chloro-12 nor-5' anhydrovinblastine | 13 | |
| Anhydrovincristine | 1,8 | 1,5 |
| Nor-5' anhydrovincristine | 0,8 | 0,66 |
| Nor-5' leurosine | 1,0 | 3 |
| Nor-5' $N_a$ desméthyl $N_a$ formyl leurosine | 2,77 | 2,5 |

(a) $I_{50}$: concentration du produit inhibant 50% de la vitesse de polymérisation de la tubuline. $I_{50}$vinblastine = $2{,}10^{-6}$.

(b) $S_{50}$: concentration du produit nécessaire pour induire 50% du maximum de spiralisation de la tubuline.

Les relations entre l'action sur la tubuline et les propriétés antitumorales sont décrites notamment dans l'article de F. Zavala et al., Experientia 34, 1497 (1978).

Le produit de l'Exemple 1 n'a pas d'activité "spiralisante" bien qu'il ait une activité semblable à la vinblastine en ce qui concerne l'inhibition de la polymérisation.

2) *Activité sur le test de la leucémie P388 de la souris*

A titre d'exemple on présente dans le Tableau II les résultats obtenus avec le composé de l'Exemple 1 sous forme de tartrate dans le traitement de la leucémie P388.

TABLEAU II

| | Sérum physiologique (témoin) | 5 mg/kg | 10 mg/kg | 25 mg/kg |
|---|---|---|---|---|
| Nombre de souris | 10 | 10 | 10 | 10 |
| Temps moyen de survie (jours) | 10,5 ± 0,8 | 13,6 ± 0,8 | 17,9 ± 0,9 | ⩾24,3 |
| I.L.S. $\mathcal{V} = \frac{T-C}{C} \times 100$ | | 29,5 | 70,5 | ⩾131,4 |
| $a = \frac{T}{C} \times 100$ | | 129,5 | 170,5 | ⩾231,4 |

T = vie moyenne (en jours) des souris du lot traité
C = vie moyenne (en jours) des souris du lot témoin souris: DBA/2 ♀
Greffe: P388 ($10^6$ cell./souris ip.)
traitement: i.p.
Délai greffe → traitement: 24 h.

3) *Activité sur le test de la leucémie L1210 de la souris*

Le tableau III résume les résultats obtenus avec le composé de l'exemple 1 sous forme de tartrate dans le traitement de la leucémie L1210.

TABLEAU III

| | Sérum physiologique (témoin) | 5 mg/kg | 10 mg/kg | 25 mg/kg |
|---|---|---|---|---|
| Nombre de souris | 10 | 10 | 10 | 10 |
| Temps moyen de survie (jours) | 8,6 ± 0,4<br>8,6 ± 0,7 | 12,0 ± 0,9 | 13,5 ± 0,9<br>12,9 ± 1,3 | 14,2 ± 0,7<br>13,5 ± 0,7 |
| I.L.S. $\frac{T-C}{C} \times 100$ | | 40 | 57<br>50 | 65<br>57 |
| $\frac{T}{C} \times 100$ | | 140 | 157<br>150 | 165<br>157 |

Souris: DBA/2
Greffe: L1210
Traitement: i.p.
Délai greffe → traitement: 24 h.
Conc: 0,5 et 1 mg/ml dans du sérum physiologique.

4) *Toxicité du produit de l'exemple 1 sous forme de tartrate*

Des souris DBA/2 ♀ reçoivent par voie intrapéritonéale en une fois une dose de produit en expérimentation et on étudie la mortalité sur 35 jours. On obtient les résultats suivants:

TABLEAU IV

| Dose (mg /kg) | Nombre de souris | Etalement de la mortalité (J) | | | | | Mortalité en 35 j |
|---|---|---|---|---|---|---|---|
| | | 1 | 3 | 4 | 6 | 9 | |
| 25 | 5 | | | | | | 0 |
| 25 | 5 | 1 | | 1 | 1 | 1 | 4/5 |
| 75 | 5 | 3 | 1 | | 1 | | 5/3 |
| 100 | 3 | | 3 | | | | 3/3 |

On observe donc une $DL_0 = 25$ mg/kg qui est à comparer avec une
$DL_{10} = 3$ mg/kg pour la vincristine,
$DL_{50} = 2$ mg/kg pour la vincristine (i. v.),
$DL_{50} = 17$ mg/kg pour la vinblastine (i. v.).

De l'ensemble de ces expériences, il résulte que le composé de l'exemple 1 est plus actif que la vincristine et la vinblastine sur les leucémies étudiées, tout en étant beaucoup moins toxique.

La présente invention concerne également les compositions pharmaceutiques contenant un nouveau composé de formule I ou un de ses sels, éventuellement en association avec tout autre produit pharmaceutique compatible, pouvant être inerte ou physiologiquement actif.

Ces compositions peuvent être présentées sous toute forme appropriée à la voie d'administration prévue. La voie parentale est la voie d'administration préférentielle, notamment la voie intraveineuse.

Les compositions selon l'invention pour administration parentérale peuvent être des solutions stériles aqueuses ou non aqueuses, des suspensions ou des émulsions. Comme solvant ou véhicule, on peut employer le propylène glycol, le polyéthylène glycol, les huiles végétales, en particulier, l'huile d'olive, les esters organiques injectables, en particulier l'oléate d'éthyle. Ces compositions peuvent également contenir des adjuvants en particulier des agents mouillants, éulsifiants et dispersants. La stérilisation peut se faire de plusieurs façons par exemple à l'aide d'un filtre bactériologique, en incorporant à la composition des agents stérilisants, par irradiation ou par chauffage. Elles peuvent être également préparées sous forme de compositions solides stériles qui peuvent être dissoutes ou dispersées au moment de l'emploi dans l'eau stérile ou tout autre milieu stérile injectable.

Les composés nouveaux ou leurs sels sont actifs dans le traitement des tumeurs solides ou liquides et plus particulièrement des cancers humains à des doses journalières comprises entre 10 et 20 mg par jour pour un adulte.

L'exemple suivant illustre une composition selon l'invention.

Exemple:

On prépare une solution contenant 10 $mg/cm_3$ de matière active en dissolvant 1 g du produit /' de l'Exemple dans 100 $cm^3$ de soluté physiologique apyrogène. La solution obtenue est répartie aseptiquement en ampoules de 2 $cm^3$ à raison de 1 $cm^3$ par ampoule. Les ampoules sont scellées et contiennent chacune 10 mg de principe actif.

**Revendications**

1. Composés de formule générale:

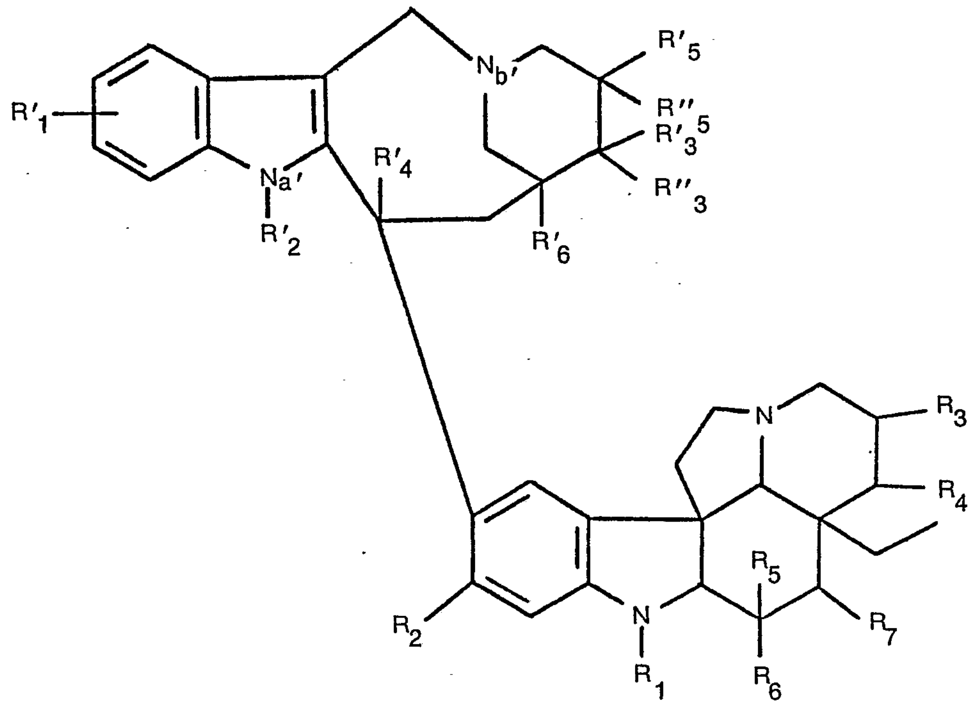

(I)

dans laquelle:

$R'_1$ représente un atome d'hydrogène ou un radical $C_1$—$C_5$ alcoxy, $C_2$—$C_5$ acyle, formyle ou halogénoacyle en $C_2$—$C_5$,

$R'_2$ représente un atome d'hydrogène, ou un radical alcoyle en $C_1$—$C_5$,

$R'_3$ et $R''_3$ identiques ou différents, représentent un atome d'hydrogène ou un radical hydroxyle, $C_2$—$C_5$ alcanoyloxyle, ou bien $R'_3$ et $R''_3$ forment ensemble un groupement carbonyle, ou bien $R'_3$ et $R'_5$ forment ensemble un pont époxy ou une double liaison,

$R'_4$ représente un atome d'hydrogène ou un radical $C_2$—$C_5$ alcoyloxy carbonyle, hydroxyméthyle, ($C_2$—$C_5$ alcanoyloxy)méthyle ou acétamido,

$R'_5$ et $R''_5$ identiques ou différents représentent un atome d'hydrogène ou un radical hydroxyle, $C_2$—$C_5$ alcanoyloxy, éthyle ou hydroxy-2-éthyle,

$R'_6$ représente un atome d'hydrogène ou un radical éthyle, hydroxy-2-éthyle ou acétyle,

$R_1$ représente un atome d'hydrogène ou un radical $C_1$—$C_5$ alcoyle, formyle ou $C_2$—$C_5$ acyle,

$R_2$ représente un atome d'hydrogène ou un radical $C_1$—$C_5$ alcoxy,

$R_3$ représente un atome d'hydrogène ou un radical hydroxyle ou $C_2$—$C_5$ alcanoyloxyle ou bien $R_3$ et $R_4$ forment ensemble un pont époxy ou une double liaison,

$R_4$ représente un atome dhydrogène ou un radical hydroxyle, $C_2$—$C_5$ alcanoyloxyle ou bien $R_4$ et $R_5$ forment ensemble un pont époxy,

$R_6$ représente un radical $C_2$—$C_5$ alcoyloxycarbonyle, hydrazido, acétamido, hydroxyméthyle ou ($C_2$—$C_5$ alcanoyloxy)méthyle,

$R_5$ et $R_7$ représentent un atome d'hydrogène ou un radical hydroxyle ou $C_2$—$C_5$ alcanoyloxyle, ainsi que leurs sels d'addition avec les acides et leurs sels d'ammonium quaternaire et les 12-chloro dérivés.

2. Composés selon la revendication 1 de formule Ia:

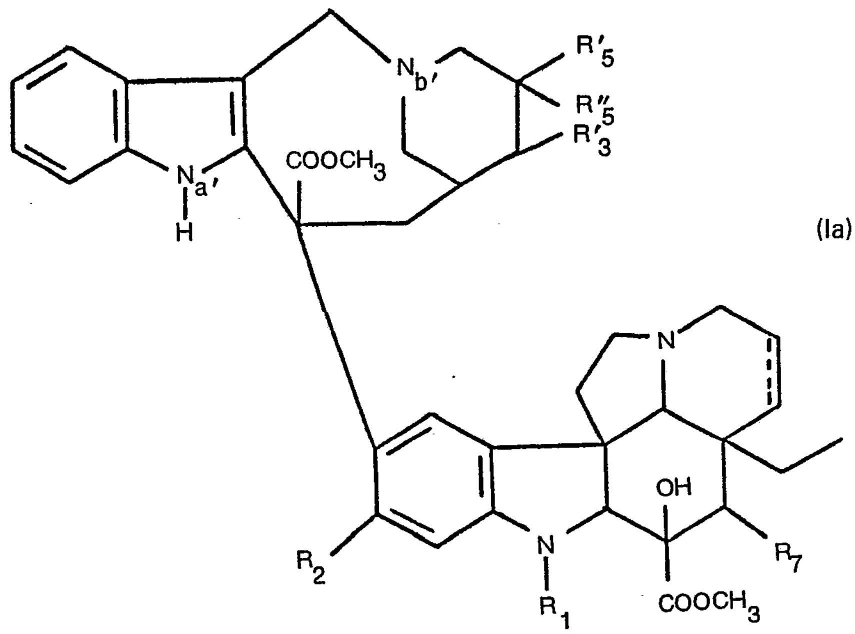

(Ia)

dans laquelle:

$R'_3$ représente un atome d'hydrogène ou un radical hydroxy,

$R'_5$ représente un atome d'hydrogène ou un radical hydroxy,

$R'_3$ et $R'_5$ représentent ensemble un pont époxy ou une double liaison,

$R''_5$ représente un atome d'hydrogène ou un radical éthyle,

$R_1$ représente un atome d'hydrogène, un radical $C_1$—$C_5$ alcoyle, formyle ou $C_2$—$C_5$ acyle,

$R_2$ représente un atome d'hydrogène ou un radical méthoxy,

$R_7$ représente un radical $C_2$—$C_5$ alcanoyloxyle,

la ligne pointillée représente une double liaison éventuelle, ainsi que les sels correspondants.

3. la nor-5' anhydrovinblastine.

4. la nor-5' anhydrovincristine.

5. la nor-5' leurosine.

6. la chloro-12 nor-5' anhydrovinblastine.

7. la nor-5' $N_a$-desméthyle $N_a$-formylleurosine.

8. Un procédé de préparation des composés selon l'une des revendications 1 à 7, caractérisé en ce que:

a) on fait réagir un composé de formule II:

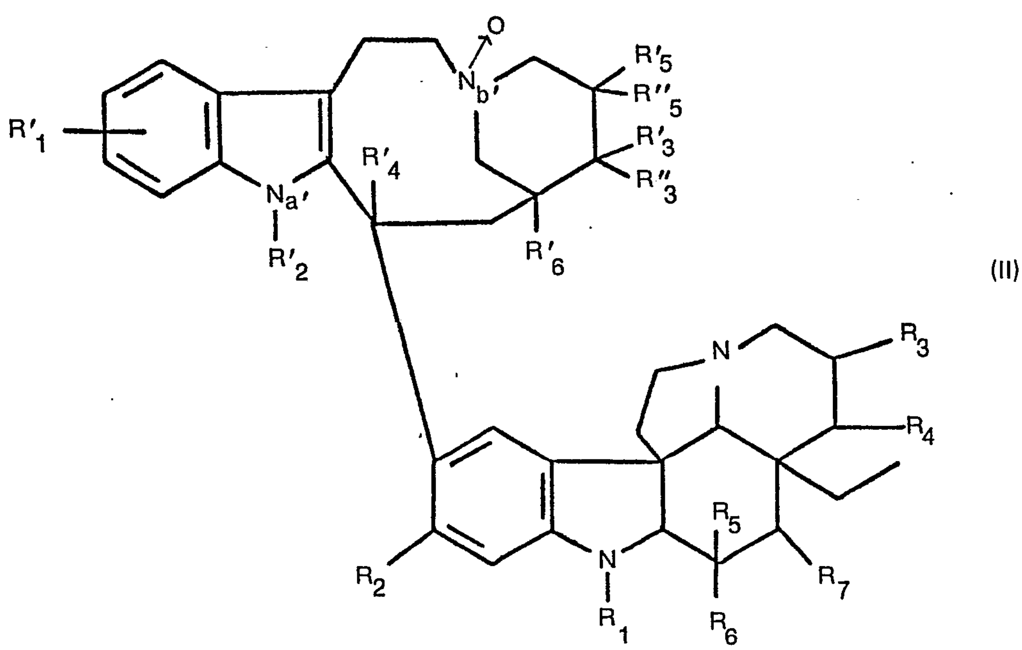

(II)

dans laquelle les substituants ont la signification donnée précédemment, en présence d'un réactif formateur d'ion immonium,

b) on traite le produit obtenu par de l'eau et on sépare le composé de formule I.

9. Procédé selon la revendication 8, caractérisé en ce que le réactif formateur d'ion immonium est un halogénure, anhydride ou sel d'acide organique ou inorganique.

10. Procédé selon la revendication 9, caractérisé en ce que le réactif formateur d'ion immonium est un halogénure, anhydride ou sel d'acide carboxylique halogéné.

11. Procédé selon la revendication 10, caractérisé en ce que le réactif formateur d'ion immonium est l'anhydride trifluoroacétique.

12. Procédé selon l'une des revendications 8 à 11, caractérisé en ce que l'étape a) est conduite dans un solvant anhydre.

13. Procédé selon la revendication 11, caractérisé en ce que le solvant anhydre est un solvant chloré.

14. Procédé selon la revendication 13, caractérisé en ce que le solvant chloré est le chlorure de méthylène, le dichloroéthane ou le chloroforme.

15. Procédé selon l'une des revendications 8 à 14, caractérisé en ce que l'étape a) est conduite à une température comprise entre −5°C et +5°C.

16. Procédé selon l'une des revendications 8 à 14, caractérisé en ce que l'étape b) est effectuée en traitant le produit de l'étape a) par un mélange de solvant organique non-nucléophile et d'eau.

17. Procédé selon la revendication 16, caractérisé en ce que le solvant organique non-nucléophile est le tétrahydrofuranne ou le dioxanne.

18. Procédé selon l'une des revendications 8 à 17, caractérisé en ce que le mélange réactionnel de l'étape a) est amené à sec avant l'étape b).

19. Procédé selon l'une des revendications 8 à 18, caractérisé en ce que le composé de formule I est séparé du mélange réactionnel de l'étape b) par extraction par un solvant chloré et chromatographie.

20. A titre de médicament nouveau un composé selon l'une des revendications 1 à 7.

21. Composition pharmaceutique contenant à titre de principe actif au moins un composé selon l'une des revendications 1 à 7.

22. Procédé de préparation de composés de formule I selon le revendication 1

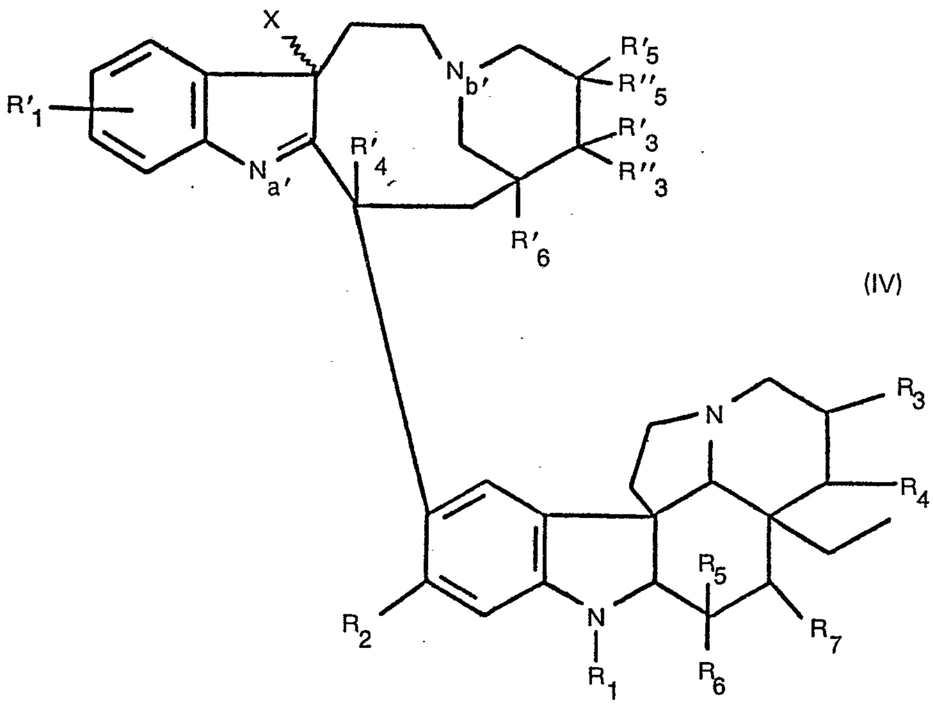

(IV)

dans laquelle les substituants ont la signification donnée précédemment et X représente un halogène, un radical hydroxy, hydroperoxy ou un $C_2$—$C_5$ alcanoyloxy; par un mélange d'eau et d'au moins un solvant organique.

23. Procédé selon la revendication 22, caractérisé en ce que le mélange d'eau et de solvant organique contient en outre des ions $Ag^+$.

24. Procédé selon la revendication 23, caractérisé en ce que les ions $Ag^+$ proviennent d'un sel d'argent choisi parmi le perchlorate, le tétrafluoroborate, le trifluoroacétate et l'acétate d'argent.

25. Procédé selon la revendication 22, caractérisé en ce que le mélange d'eau et de solvant organique contient en outre un réactif acide.

26. Procédé selon l'une des revendications 22 à 25, caractérisé en ce que le solvant organique utilisé est choisi parmi le tétrahydrofuranne et le dioxanne.

27. Procédé selon l'une des revendications 22 à 25, caractérisé en ce que le traitement est conduit à une température comprise entre 0°C et 70°C.

28. Procédé selon l'une des revendications 22 à 27, caractérisé en ce que le composé de formule I est extrait du mélange réactionnel à l'aide d'un solvant chloré.

29. Procédé selon l'une des revendications 22 à 28, caractérisé en ce que le composé de formule IV dans lequel X est un halogène est préparé par halogénation d'un composé de formule III:

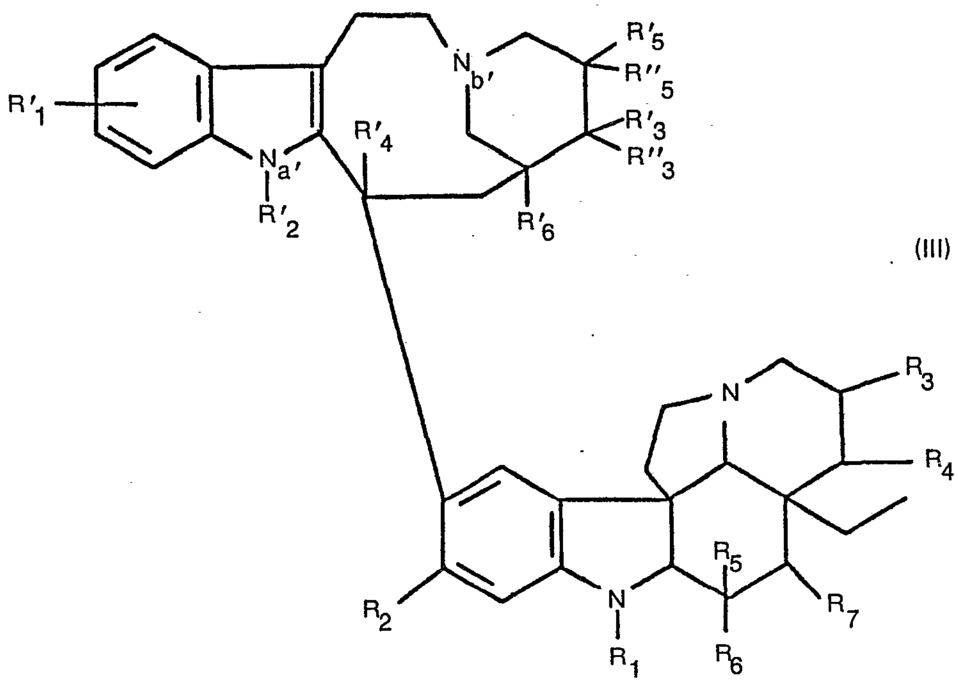

(III)

dans laquelle les différents substituants ont la signification donnée précédemment, par addition d'un réactif générateur d'ion halogène positif dans un solvant organique.

30. Procédé selon la revendication 29, caractérisé en ce que le réactif générateur d'ion halogène positif est choisi parmi les N-halogénobenzotriazoles, les hypohalogénites, les N-halogénoamides ou N-halogénoimides.

31. Procédé selon l'une des revendications 29 et 30, caractérisé en ce que le solvant organique utilisé est choisi parmi le benzène, le chloroforme, le dichlorométhane, l'acétonitrile, le tétrahydrofuranne et le dioxanne.

32. Procédé selon l'une des revendications 28 à 31, caractérisé en ce que la réaction d'halogénation est conduite à une température comprise entre —10°C et +20°C.

33. Procédé selon l'une des revendications 28 à 32, caractérisé en ce que le composé de formule II est isolé du mélange réactionnel par extraction et évaporation avant d'être traité par le mélange d'eau et d'au moins un solvant organique.

34. Procédé selon l'une des revendications 28 à 32, caractérisé en ce que le composé de formule II est traité par le mélange d'eau et d'au moins un solvant organique sans être isolé du mélange réactionnel d'halogénation.

35. Procédé selon l'une des revendications 28 à 32 et 17, caractérisé en ce que l'halogénation est conduite en présence d'eau et que l'on obtient directement le composé de formule I.

36. Procédé selon la revendication 35, caractérisé en ce que l'halogénation est conduite en présence d'eau et d'ion $Ag^+$ ou d'un réactif acide et que l'on obtient directement le composé de formule I.

37. Procédé selon l'une des revendications 28 à 36, caractérisé en ce que pour préparer des composés de formule IV et de formule I'on utilise une quantité stoechiométrique de réactif générateur d'ion halogène positif.

38. Procédé selon l'une des revendications 22 à 26, caractérisé en ce que le composé de formule IV dans lequel X est un radical hydroperoxyde est préparé par oxydation d'un composé de formule III:

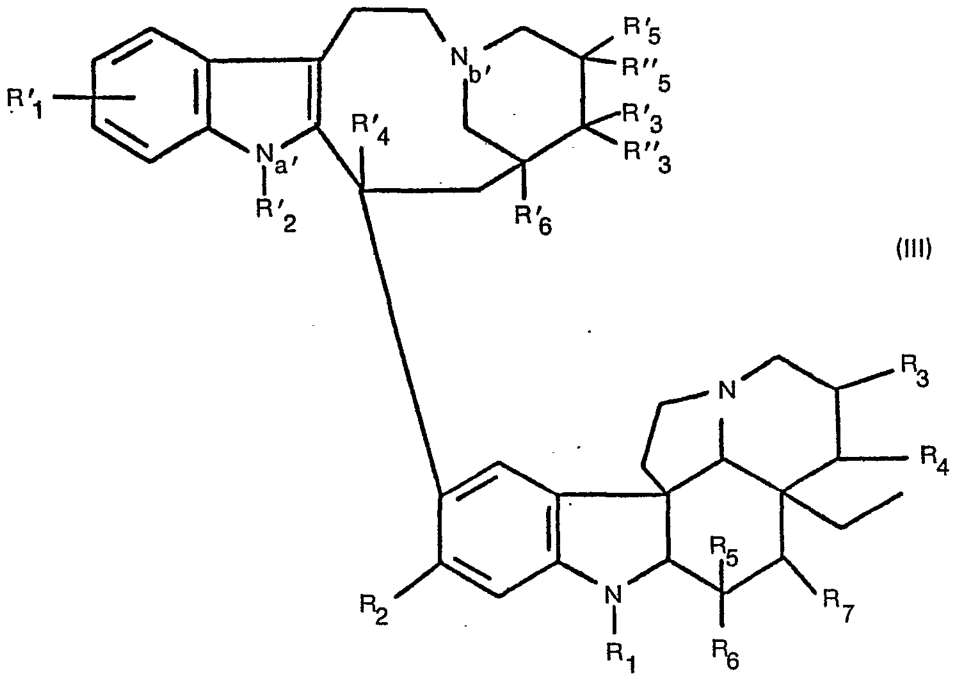

(III)

dans laquelle les différents substituants ont la signification donnée précédemment, à l'aide d'oxygène en présence d'un sel métallique.

39. Procédé selon la revendication 38, caractérisé en ce que le composé de formule IV obtenu est réduit par un trialcoyle ou triaryle phosphite afin de donner un composé de formule IV dans lequel X est le radical hydroxy.

## Claims

1. Compounds corresponding to the general formula:

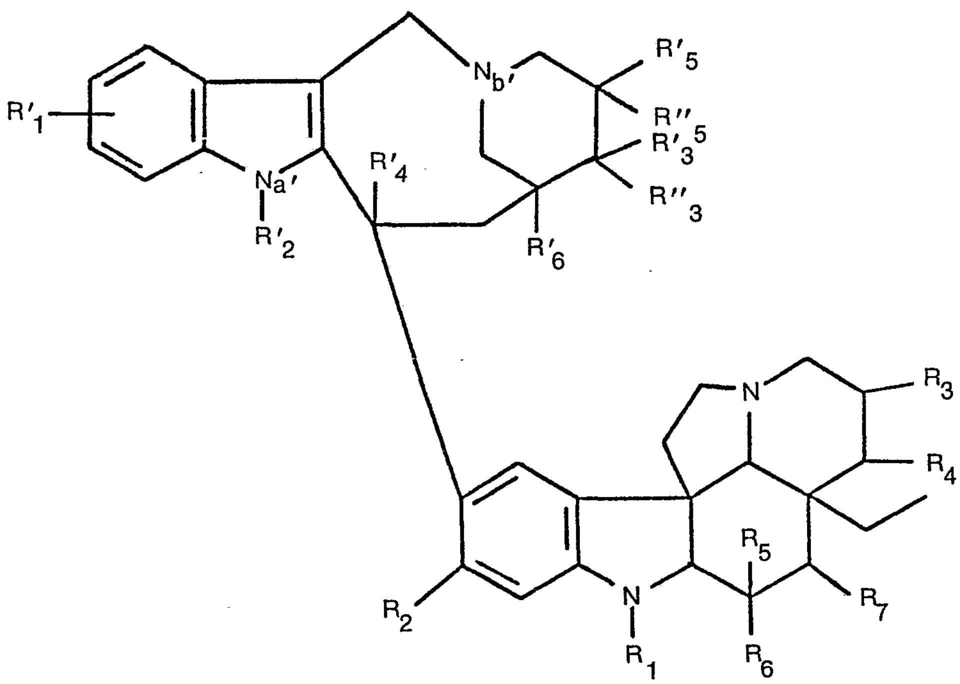

(I)

wherein:

R'$_1$ represents a hydrogen atom or a $C_1$—$C_5$ alkoxy, $C_2$—$C_5$ acyl, formyl or $C_2$—$C_5$ halo-acyl radical,

$R'_2$ represents a hydrogen atom or a $C_1$—$C_5$ alkyl radical,

$R'_3$ and $R''_3$ which may be the same or different, represent a hydrogen atom or a hydroxyl or a $C_2$—$C_5$ alkanoyloxyl radical, or $R'_3$ and $R''_3$ together form a carbonyl group, or $R'_3$ and $R'_5$ together form an epoxy bridge or a double bond,

$R'_4$ represents a hydrogen atom or a $C_2$—$C_5$ alkyloxy carbonyl, hydroxymethyl, ($C_2$—$C_5$ alkanoyloxy) methyl or acetamido radical,

$R'_5$ and $R''_5$ which may be the same or different, represent a hydrogen atom or a hydroxyl, $C_2$—$C_5$ alkanoyloxy, ethyl or 2-hydroxyethyl radical,

$R'_6$ represents a hydrogen atom or an ethyl, 2-hydroxy ethyl or acetyl radical,

$R_1$ represents a hydrogen atom or a $C_1$—$C_5$ alkyl, formyl or $C_2$—$C_5$ acyl radical,

$R_2$ represents a hydrogen atom or a $C_1$—$C_5$ alkoxy radical,

$R_3$ represents a hydrogen atom or a hydroxyl or a $C_2$—$C_5$ alkanoyloxyl radical, or $R_3$ and $R_4$ together form an epoxy bridge or a double bond,

$R_4$ represents a hydrogen atom or a hydroxyl or a $C_2$—$C_5$ alkanoyloxyl radical, or $R_4$ and $R_5$ together form an epoxy bridge,

$R_6$ represents a $C_2$—$C_5$ alkyloxy carbonyl, hydrazido, acetamido, hydroxymethyl or ($C_2$—$C_5$ alkanoyloxy)methyl radical, and

$R_5$ and $R_7$ represent a hydrogen atom or a hydroxyl or a $C_2$—$C_5$ alkanoyloxyl radical,

and the addition salts thereof with acids and the quaternary ammonium salts thereof and 12-chloro derivatives.

2. Compounds according to claim 1 corresponding to the formula Ia:

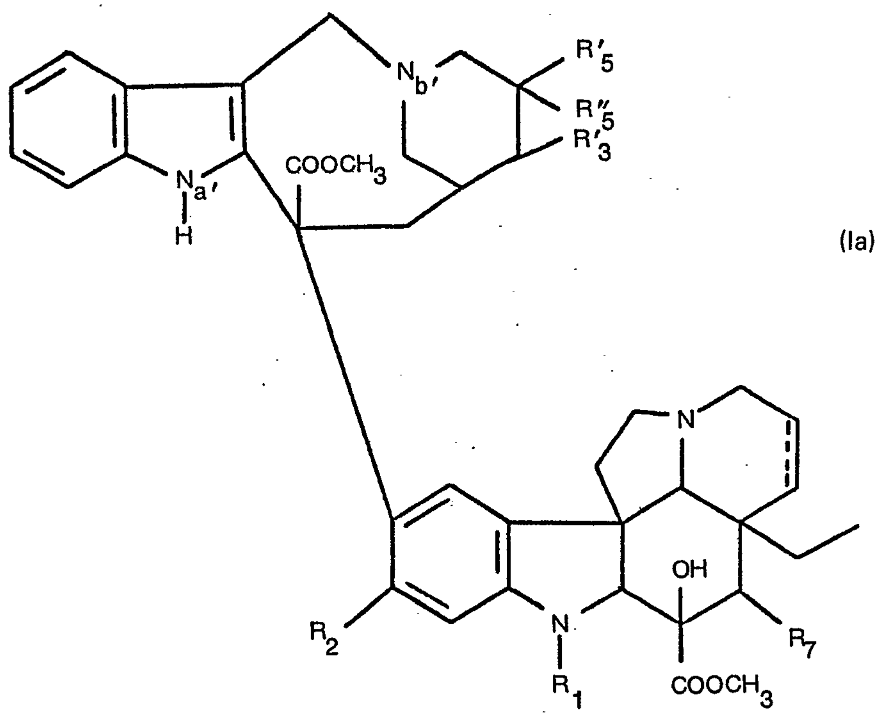

(Ia)

wherein:

$R'_3$ represents a hydrogen atom or a hydroxy radical,

$R'_5$ represents a hydrogen atom or a hydroxy radical,

$R'_3$ and $R'_5$ together represent an epoxy bridge or a double bond,

$R''_5$ represents a hydrogen atom or an ethyl radical,

$R_1$ represents a hydrogen atom, a $C_1$—$C_5$ alkyl, formyl or $C_2$—$C_5$ acyl radical,

$R_2$ represents a hydrogen atom or a methoxy radical, and

$R_7$ represents a $C_2$—$C_5$ alkanoyloxyl radical,

the dotted line represents a possible double bond, and the corresponding salts.

3. 5′-nor-anhydrovinblastine.

4. 5′-nor-anhydrovincristine.

5. 5′-nor leurosine.

6. 12-chloro-5′-nor-anhydrovinblastine.

7. 5′-nor-$N_a$-desmethyl $N_a$-formylleurosine.

8. A process for the preparation of compounds according to one of claims 1 to 7, characterised in that:

(a) a compound corresponding to formula II is reacted:

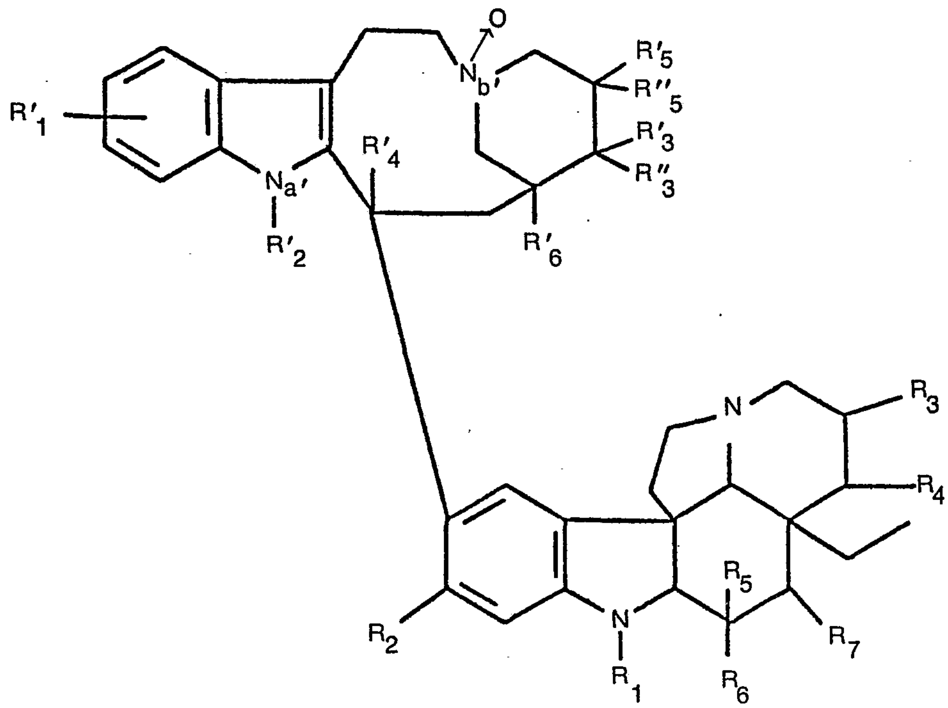

(II)

wherein the substituents are as previously defined, in the presence of an immonium ion-forming reagent,

(b) the product which is obtained is treated with water and the compound corresponding to formula I is separated.

9. A process according to claim 8, characterised in that the immonium ion-forming reagent is a halide, an anhydride or an organic or inorganic acid salt.

10. A process according to claim 9, characterised in that the immonium ion-forming reagent is a halide, an anhydride or a halogenated carboxylic acid salt.

11. A process according to claim 10, characterised in that the immonium ion-forming reagent is trifluoro-acetic anhydride.

12. A process according to one of claims 8 to 11, characterised in that stage a) is carried out in an anhydrous solvent.

13. A process according to claim 11, characterised in that the anhydrous solvent is a chlorinated solvent.

14. A process according to claim 13, characterised in that the chlorinated solvent is methylene chloride, dichloroethane or chloroform.

15. A process according to one of claims 8 to 14, characterised in that stage a) is carried out at a temperature of from −5 to +5°C.

16. A process according to one of claims 8 to 14, characterised in that stage b) is carried out by treating the product of stage a) with a mixture of non-nucleophilic organic solvent and water.

17. A process according to claim 16, characterised in that the non-nucleophilic organic solvent is tetrahydrofuran or dioxane.

18. A process according to one of claims 8 to 17, characterised in that the reaction mixture of stage a) is dried before stage b).

19. A process according to one of claims 8 to 18, characterised in that the compound corresponding to formula I is separated from the reaction mixture of stage b) by extraction using a chlorinated solvent and by means of chromatography.

20. A compound according to one of claims 1 to 7 as a new medicament.

21. A pharmaceutical composition containing as active principle at least one compound according to one of claims 1 to 7.

22. A process for the preparation of compounds corresponding to formula I according to claim 1 in which a compound corresponding to formula IV:

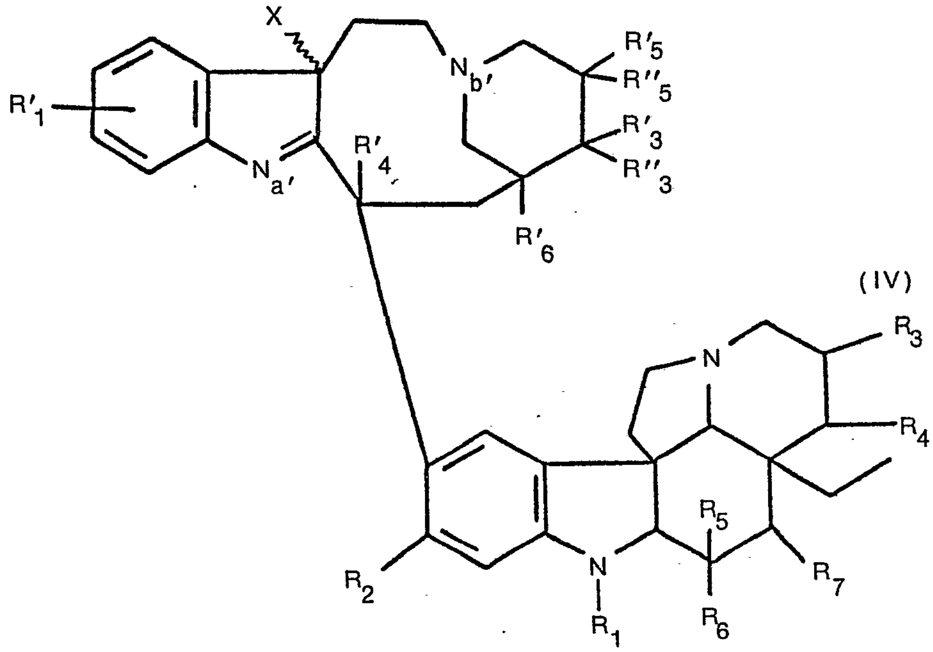

(IV)

wherein the substituents are as previously defined, and X represents a halogen, a hydroxy, hydroperoxy or a $C_2$—$C_5$ alkanoyloxy radical; is treated with a mixture of water and at least one organic solvent.

23. A process according to claim 22, characterised in that the mixture of water and organic solvent also contains $Ag^+$ ions.

24. A process according to claim 23, characterised in that the $Ag^+$ ions originate from a silver salt selected from among perchlorate, tetrafluoroborate, trifluoroacetate and acetate of silver.

25. A process according to claim 22, characterised in that the mixture of water and organic solvent also contains an acid reagent.

26. A process according to one of claims 22 to 25, characterised in that the organic solvent which is used is selected from among tetrahydrofuran and dioxane.

27. A process according to one of claims 22 to 25, characterised in that the treatment is carried out at a temperature of from 0 to 70°C.

28. A process according to one of claims 22 to 27, characterised in that the compound corresponding to formula I is extracted from the reaction mixture using a chlorinated solvent.

29. A process according to one of claims 22 to 28, characterised in that the compound corresponding to formula IV, wherein X represents a halogen, is prepared by the halogenation of a compound corresponding to formula III:

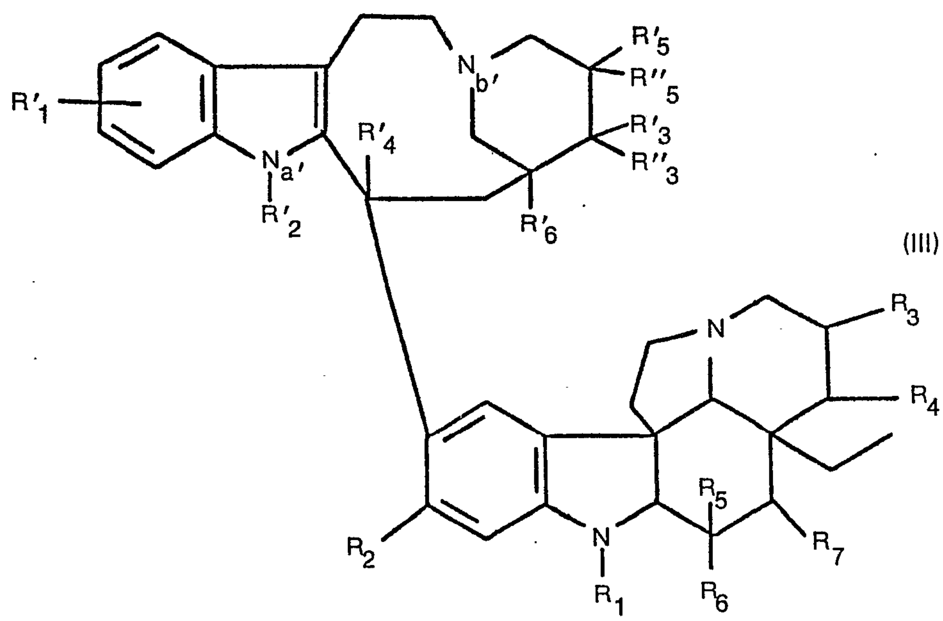

(III)

wherein the different substituents are as previously defined, by the addition of a positive halogen ion-generating reagent in an organic solvent.

30. A process according to claim 29, characterised in that the positive halogen ion-generating reagent is selected from among N-halo-benzotriazoles, hypohalogenites, N-halo-amides or N-halo-imides.

31. A process according to one of claims 29 and 30, characterised in that the organic solvent which is used is selected from among benzene, chloroform, dichloromethane, acetonitrile, tetrahydrofuran and dioxane.

32. A process according to one of claims 28 to 31, characterised in that the halogenation reaction is carried out at a temperature of from −10 to +20°C.

33. A process according to one of claims 28 to 32, characterised in that the compound corresponding to formula II is isolated from the reaction mixture by extraction and evaporation before being treated with the mixture of water and at least one organic solvent.

34. A process according to one of claims 28 to 32, characterised in that the compound corresponding to formula II is treated with the mixture of water and at least one organic solvent without being isolated from the halogenation reaction mixture.

35. A process according to one of claims 28 to 32 and 17, characterised in that halogenation is carried out in the presence of water, and the compound corresponding to formula I is directly obtained.

36. A process according to claim 35, characterised in that halogenation is carried out in the presence of water and $Ag^+$ ion or an acid reagent, and the compound corresponding to formula I is directly obtained.

37. A process according to one of claims 28 to 36, characterised in that for the preparation of compounds corresponding to formule IV and to formula I, a stoichiometric quantity of positive halogen ion-generating reagent is used.

38. A process according to one of claims 22 to 26, characterised in that the compound corresponding to formula IV, wherein X represents a hydroperoxide radical is prepared by the oxidation of a compound corresponding to formula III:

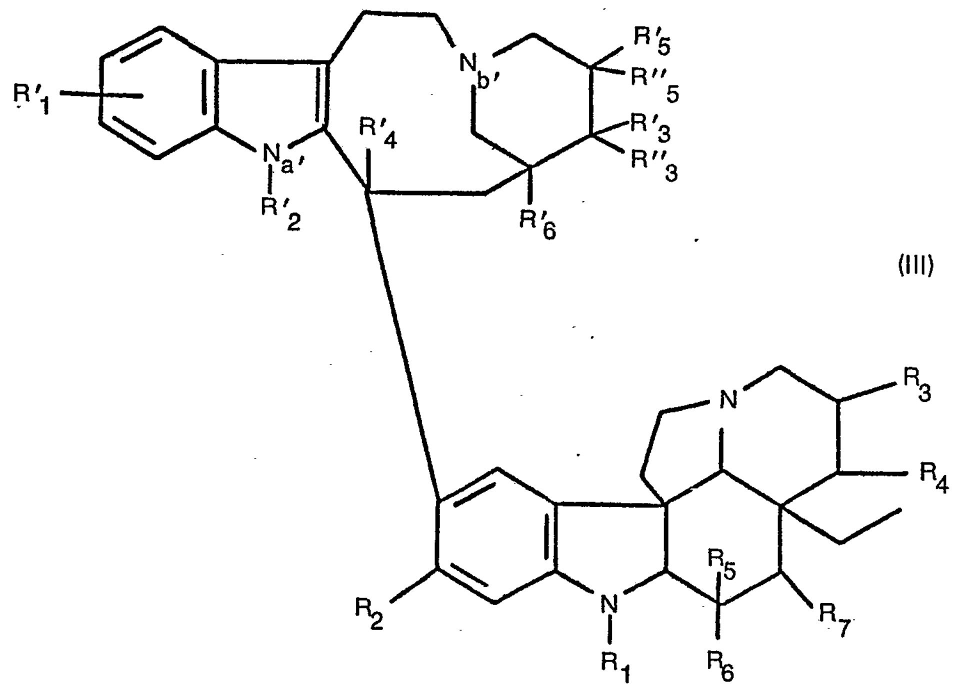

(III)

wherein the different substituents are as previously defined, using oxygen in the presence of a metallic salt.

39. A process according to claim 38, characterised in that the compound corresponding to formula IV which is obtained is reduced by a trialkyl or triaryl phosphite in order to produce a compound corresponding to formula IV, wherein X represents the hydroxy radical.

## Patentansprüche

1. Verbindungen der allgemeinen Formel

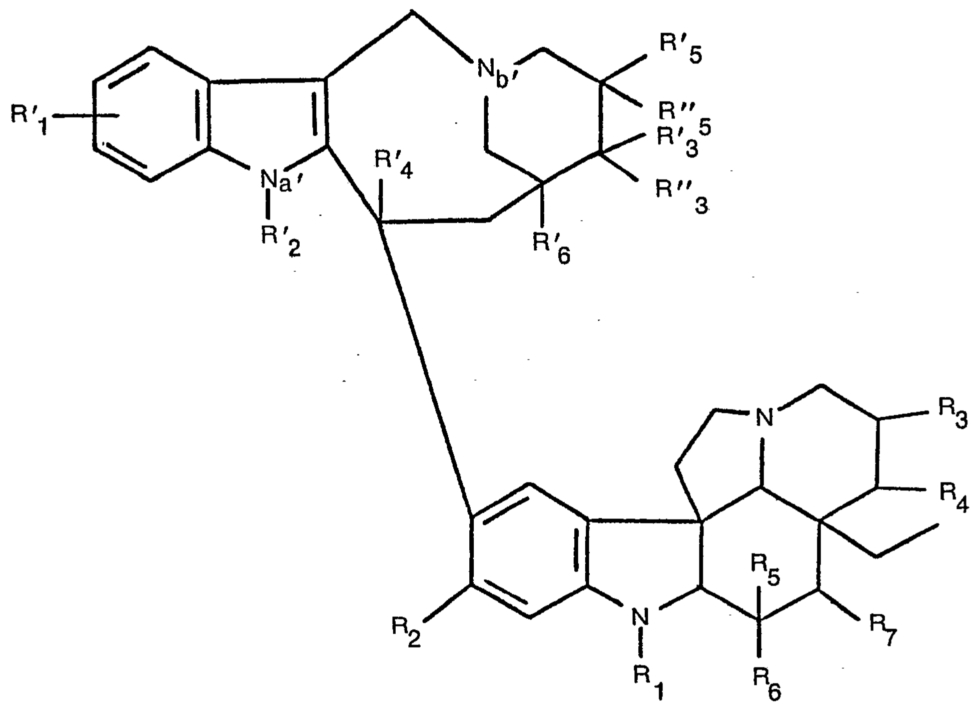

(I)

in der:

$R'_1$ für Wasserstoff oder eine Alkoxygruppe mit 1 bis 5 Kohlenstoffatomen, eine Acylgruppe mit 2 bis 5 Kohlenstoffatomen, Formyl- oder Halogenacyl mit 2 bis 5 Kohlenstoffatomen steht,

$R'_2$ für Wasserstoff oder eine Alkylgruppe mit 1 bis 5 Kohlenstoffatomen steht,

$R'_3$ und gleich oder verschieden sind und für Wasserstoff

$R''_3$ oder eine Hydroxylgruppe, einer Alkanoyloxygruppe mit 2 bis 5 Kohlenstoffatomen stehen oder $R'_3$ und $R''_3$ gemeinsam eine Carbonylgruppe, oder $R'_3$ und $R'_5$ gemeinsam eine Epoxybrücke oder eine Doppelbindung bilden,

$R'_4$ für Wasserstoff oder eine $C_2$—$C_5$ Alkoxycarbonylgruppe, Hydroxymethylgruppe, ($C_2$—$C_5$ Alkanoyloxy) methyl- oder Acetamidogruppe steht,

$R'_5$ und gleich oder verschieden sind und für Wasserstoff

$R''_5$ oder eine Hydroxylgruppe, Alkanoloxygruppe mit 2 bis 5 Kohlenstoffatomen, Äthyl- oder 2-Hydroxyäthylgruppe stehen,

$R'_6$ für Wasserstoff oder ein Äthyl-, 2-Hydroxyäthyl- oder Acetylgruppe steht,

$R_1$ für Wasserstoff oder eine Alkylgruppe mit 1 bis 5 Kohlenstoffatomen, Formylgruppe oder Acylgruppe mit 2 bis 5 Kohlenstoffatomen steht,

$R_2$ für Wasserstoff oder eine Alkoxygruppe mit 1 bis 5 Kohlenstoffatomen steht,

$R_3$ für Wasserstoff oder eine Hydroxylgruppe oder eine Alkanoyloxygruppe mit 2 bis 5 Kohlenstoffatomen steht, oder $R_3$ und $R_4$ gemeinsame eine Epoxybrücke oder eine Doppelbindung bilden,

$R_4$ für Wasserstoff oder eine Hydroxylgruppe, Alkanoyloxygruppe mit 2 bis 5 Kohlenstoffatomen steht, oder $R_4$ und $R_5$ gemeinsam eine Epoxybrücke bilden,

$R_6$ für eine Alkoxycarbonylgruppe mit 2 bis 5 Kohlenstoffatomen, Hydrazido-, Acetamido-, Hydroxymethyl- oder ($C_2$—$C_5$ Alkanoyloxy)methylgruppe steht,

$R_5$ und $R_7$ für Wasserstoff oder eine Hydroxyl- oder $C_2$—$C_5$ Alkanoyloxygruppe stehen, sowie deren Additionssalze mit Säuren und ihre quaternären Ammoniumsalze sowie die 12-Chlorderivate.

2. Verbindung nach Anspruch 1 gemäß Formel 1a:

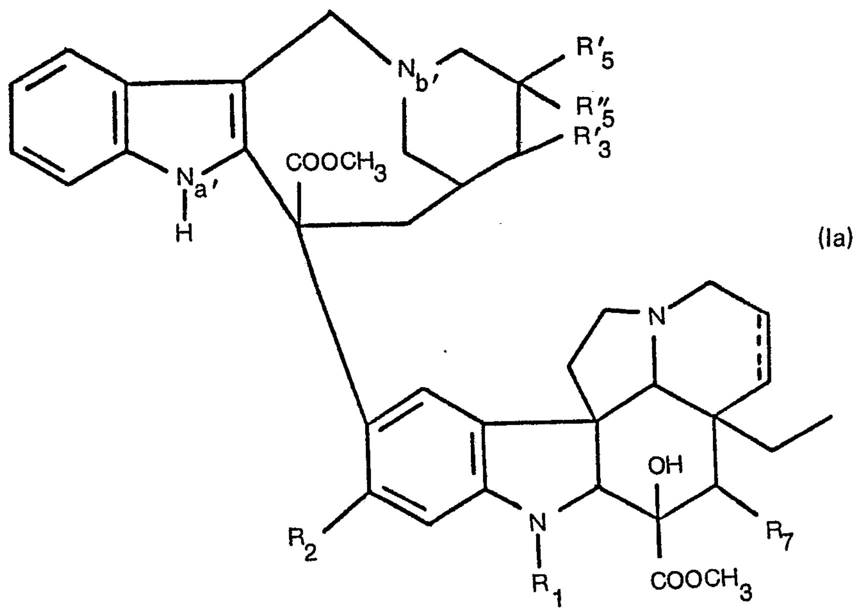

(Ia)

in der

$R'_3$ für Wasserstoff oder eine Hydroxygruppe steht,
$R'_5$ für Wasserstoff oder eine Hydroxygruppe steht,
$R'_3$ und $R'_5$ gemeinsam eine Epoxybrücke oder eine Doppelbindung bilden,
$R''_5$ für Wasserstoff oder eine Äthylgruppe steht,
$R_1$ für Wasserstoff, eine Alkylgruppe mit 1 bis 5 Kohlenstoffatomen, Formyl- oder Acylgruppe mit 2 bis 5 Kohlenstoffatomen steht,
$R_2$ für Wasserstoff oder eine Methoxygruppe steht,
$R_7$ für eine $C_2$—$C_5$ Alkanoyloxygruppe steht,

wobei die gepunktete Linie für eine mögliche Doppelbindung steht, ebenso wie die entsprechenden Salze.

3. 5'-Noranhydrovinblastin.
4. 5'-Noranhydrovincristin.
5. 5'-Norleurosin.
6. 12-Chlor-5'-noranhydrovinblastin.
7. 5'-Nor $N_a$-desmethyl $N_a$-formylleurosin.
8. Verfahren zur Herstellung der Verbindungen gemäß einem der Ansprüche 1 bis 7, dadurch gekennzeichnet, daß

a) man eine Verbindung gemäß Formel II

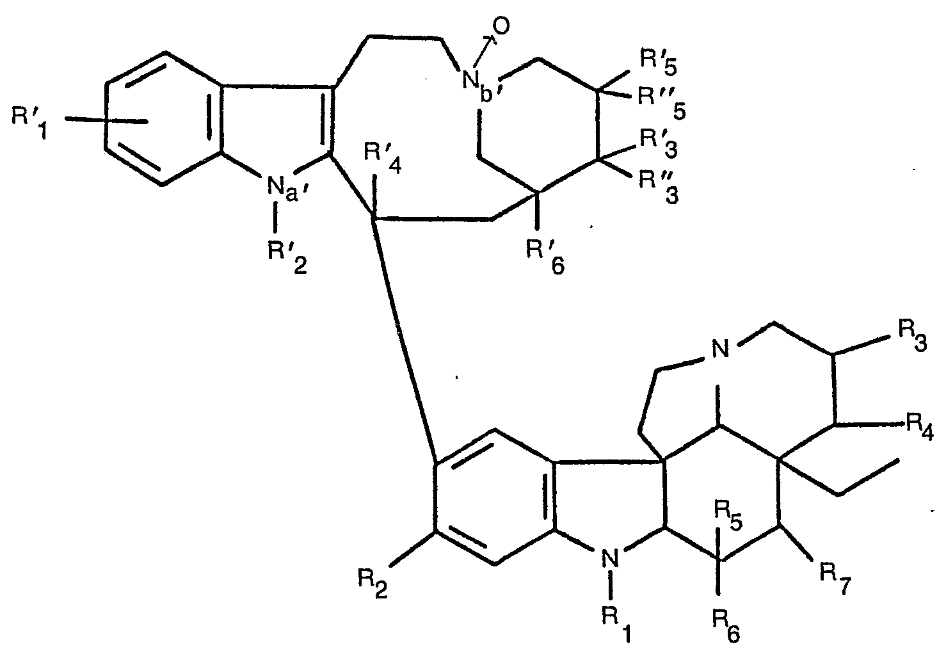

(II)

in der die Substituenten die vorhergehende Bedeutung besitzen, in Gegenwart eines Reaktiven Immoniumionbildners umsetzt,

b) man das erhaltene Produkt mit Wasser behandelt und die Verbindung gemäß Formel I abtrennt.

9. Verfahren nach Anspruch 8, dadurch gekennzeichnet, daß der reaktive Immoniumionbildner ein Halogenid, Anhydrid oder Salz einer organischen oder anorganischen Säure ist.

10. Verfahren nach Anspruch 9, dadurch gekennzeichnet, daß der reaktive Immoniumionbildner ein Halogenid, Anhydrid oder Salz einer halogenierten Carbonsäure ist.

11. Verfahren nach Anspruch 10, dadurch gekennzeichnet, daß der reaktive Immoniumionbildner Trifluoressigsäureanhydid ist.

12. Verfahren nach einem der Ansprüche 8 bis 11, dadurch gekennzeichnet, daß die Stufe a) in wasserfreiem Lösungsmittel durchgeführt wird.

13. Verfahren nach Anspruch 11, dadurch gekennzeichnet, daß das wasserfreie Lösungsmittel ein chloriertes Lösungsmittel ist.

14. Verfahren nach Anspruch 13, dadurch gekennzeichnet, daß das chlorierte Lösungsmittel Methylenchlorid, Dichloräthan oder Chloroform ist.

15. Verfahren nach einem der Ansprüche 8 bis 14, dadurch gekennzeichnet, daß die Stufe a) bei einer Temperatur zwischen −5°C und +5°C durchgeführt wird.

16. Verfahren nach einem der Ansprüche 8 bis 14, dadurch gekennzeichnet, daß die Stufe b) durchgeführt wird, indem man das Produkt der Stufe a) mit einer Mischung aus Wasser und einem organischen, nichtnukleophilen Lösungsmittel behandelt.

17. Verfahren nach Anspruch 16, dadurch gekennzeichnet, daß das organische nicht-nukleophile Lösungsmittel Tetrahydrofuran oder Dioxan ist.

18. Verfahren nach einem der Ansprüche 8 bis 17, dadurch gekennzeichnet, daß die Reaktionsmischung der Stufe a) vor Stufe b) getrocknet wird.

19. Verfahren nach einem der Ansprüche 8 bis 18, dadurch gekennzeichnet, daß die Verbindung der Formel I aus der Reaktionsmischung der Stufe b) durch Extraktion mit einem chlorierten Lösungsmittel abgetrennt und chromatographiert wird.

20. Verbindung gemäß einem der Ansprüche 1 bis 7 als neues Arzneimittel.

21. Pharmazeutische Zusammensetzung mit Gehalt an mindestens einer Verbindung gemäß einem der Ansprüche 1 bis 7 als Wirkstoff.

22. Verfahren zur Herstellung von Verbindungen gemäß Formel I nach Anspruch 1, dadurch gekennzeichnet, daß man eine Verbindung der Formel IV

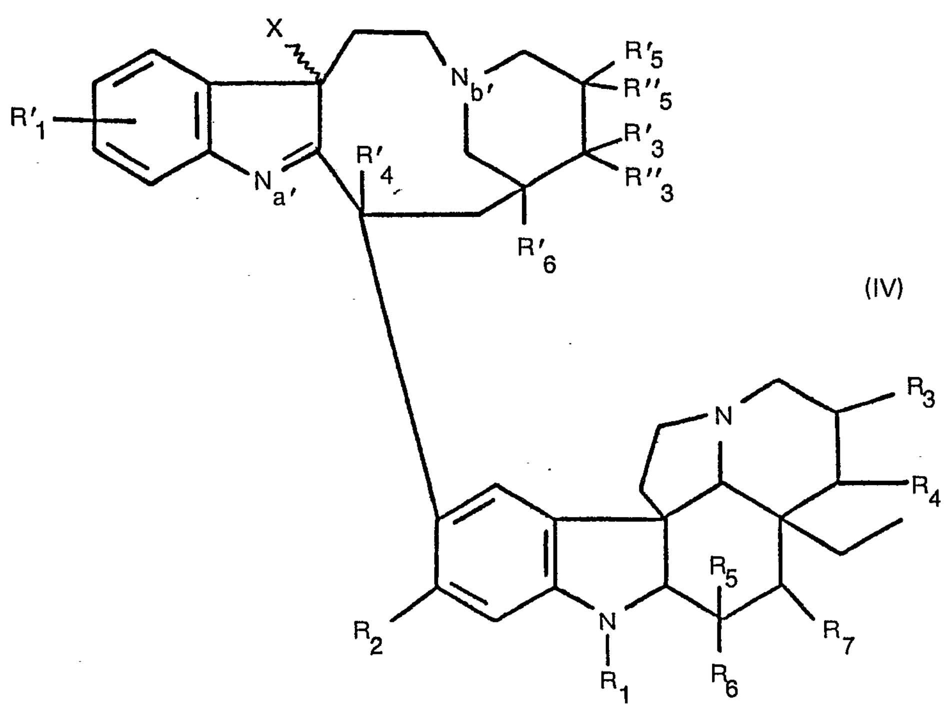

(IV)

in der die Substituenten die zuvor gegebene Bedeutung besitzen und X für ein Halogen, eine Hydroxy-, Hydroperoxy- oder eine $C_2$—$C_5$-Alkanoyloxygruppe steht, mit einer Mischung aus Wasser und mindestens einem organischen Lösungsmittel behandelt.

23. Verfahren nach Anspruch 22, dadurch gekennzeichnet, daß die Mischung aus Wasser und organischem Lösungsmittel zusätzlich Silber ($Ag^+$) ionen enthält.

24. Verfahren nach Anspruch 23, dadurch gekennzeichnet, daß die Silber ($Ag^+$) ionen aus einem Silbersalz, nämlich einem Perchlorat, Tetrafluorborat, Trifluoracetat oder Acetat des Silbers stammen.

25. Verfahren nach Anspruch 22, dadurch gekennzeichnet, daß die Mischung aus Wasser und organischem Lösungsmittel zusätzlich ein saures Reagenz enthält.

26. Verfahren nach einem der Ansprüch 22 bis 25, dadurch gekennzeichnet, daß das verwendete organische Lösungsmittel Tetrahydrofuran oder Dioxan ist.

27. Verfahren nach einem der Ansprüche 22 bis 25, dadurch gekennzeichnet, daß die Behandlung bei einer Temperatur zwischen 0°C und 70°C durchgeführt wird.

28. Verfahren nach einem der Ansprüche 22 bis 27, dadurch gekennzeichnet, daß die Verbindung gemäß Formel I aus der Reaktionsmischung mittels eines chlorierten Lösungsmittels extrahiert wird.

29. Verfahren nach einem der Ansprüche 22 bis 28, dadurch gekennzeichnet, daß die Verbindung gemäß Formel IV, in der X ein Halogen ist, durch Halogenierung einer Verbindung gemäß Formel III

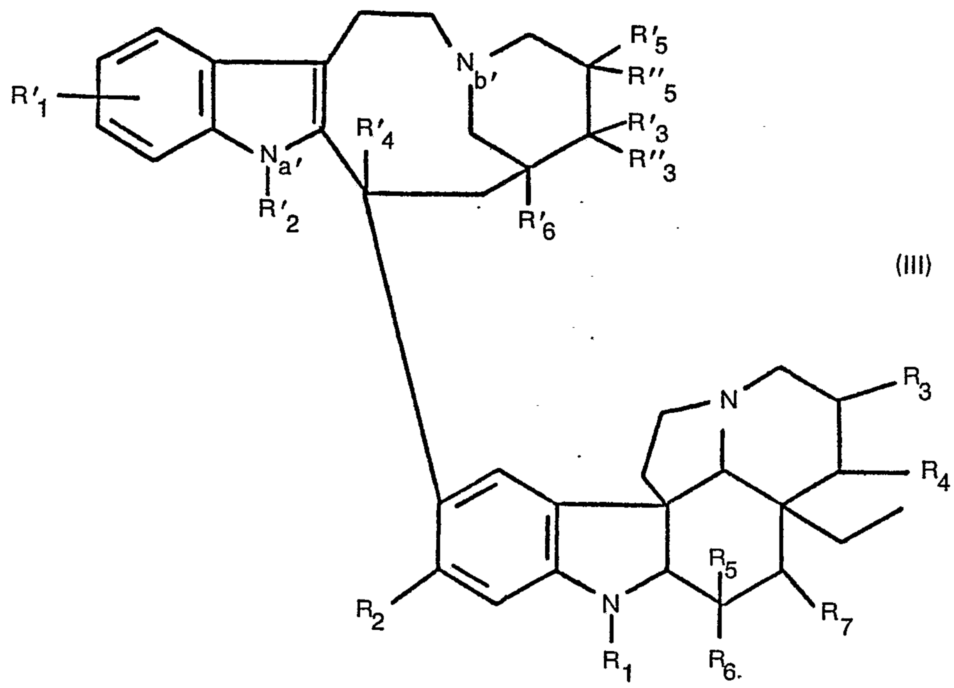

(III)

in der die verschiedenen Substituenten die vorgegebene Bedeutung besitzen, hergestellt wird, wobei ein reaktiver Erzeuger von pisitiven Halogenionen in einem organischen Lösungsmittel zugesetzt wird.

30. Verfahren nach Anspruch 29, dadurch gekennzeichnet, daß der reaktive Erzeuger für positive Halogenionen N-Halogenbenzotrazole, Hypohalogenite, N-Halogenamide oder N-Halogenimide sind.

31. Verfahren nach einem der Ansprüche 29 und 30, dadurch gekennzeichnet, daß als organisches Lösungsmittel Benzol, Chloroform, Dichlormethan, Acetonitril, Tetrahydrofuran oder Dioxan verwendet wird.

32. Verfahren nach einem der Ansprüche 28 bis 31, dadurch gekennzeichnet, daß die Halogenierung bei einer Temperatur zwischen —10°C und +20°C durchgeführt wird.

33. Verfahren nach einem der Ansprüche 28 bis 32, dadurch gekennzeichnet, daß die Verbindung gemäß Formel II aus der Reaktionsmischung durch Extraktion und Verdampfung vor der Behandlung mit der Mischung aus Wasser und mindestens einem organischen Lösungsmittel isoliert wird.

34. Verfahren nach einem der Ansprüche 28 bis 32, dadurch gekennzeichnet, daß die Verbindung gemäß Formel II mit einer Mischung aus wasser und mindestens einem organischen Lösungsmittel ohne Isolierung aus der Reaktionsmischung der Halogenierung behandelt wird.

35. Verfahren nach einem der Ansprüche 28 bis 32 und 17, dadurch gekennzeichnet, daß die Halogenierung in Gegenwart von Wasser durchgeführt wird, und daß man direkt die Verbindung gemäß Formel I erhält.

36. Verfahren nach Anspruch 35, dadurch gekennzeichnet, daß die Halogenierung in Gegenwart von Wasser und Silber ($Ag^+$) ionen oder einem sauren Regenz durchgeführt wird, und daß man direkt die Verbindung gemäß Formel I erhält.

37. Verfahren nach einem der Ansprüche 28 bis 36, dadurch gekennzeichnet, daß man zur Herstellung von Verbindungen der Formel IV und der Formel I eine stöchiometrische Menge des reaktiven Erzeugers von positiven Halogenionen verwendet.

38. Verfahren nach einem der Ansprüche 22 bis 26, dadurch gekennzeichnet, daß die Verbindung gemäß Formel IV, in der X eine Hydroperoxidgruppe ist, durch Oxidation einer Verbindung gemäß Formel III

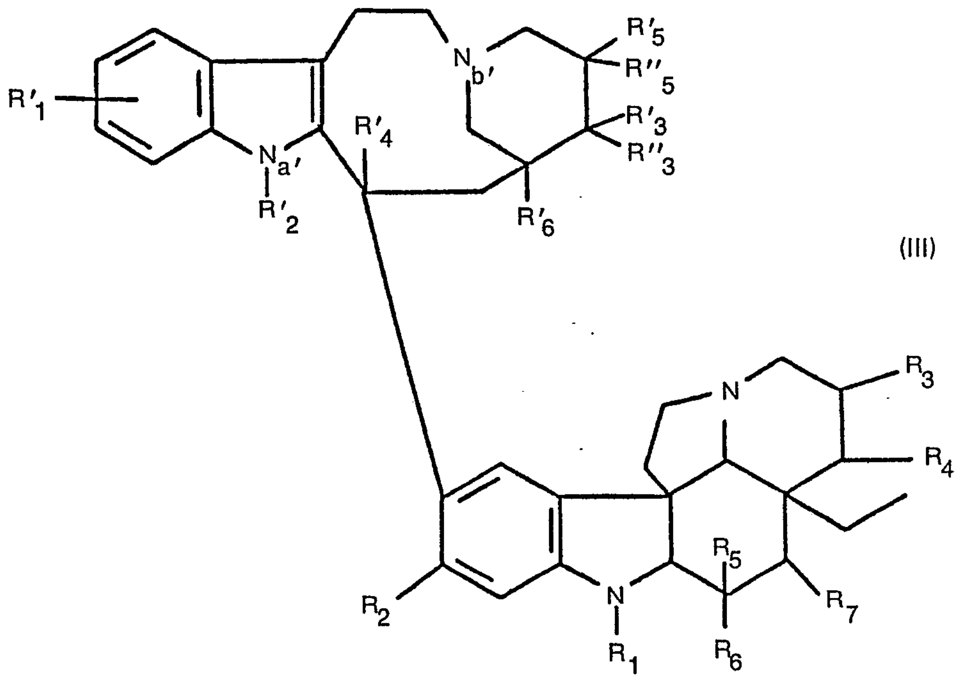

(III)

hergestellt wird, wobei die verschiedenen Substituenten die vorgegebene Bedeutung besitzen, wobei die Oxidation mittels Sauerstoff in Gegenwart eines Metallsalzes durchgeführt wird.

39. Verfahren nach Anspruch 38, dadurch gekennzeichnet, daß die erhaltene Verbindung gemäß Formel IV mit einem Trialkyl- oder Triarylphosphit reduziert wird, um eine Verbindung gemäß Formel IV, in der X die Hydroxygruppe ist, zu erhalten.